(19) Europäisches Patentamt
European Patent Office
Office européen des brevets

(11) **EP 4 534 020 A1**

(12) **EUROPEAN PATENT APPLICATION**
published in accordance with Art. 153(4) EPC

(43) Date of publication:
09.04.2025 Bulletin 2025/15

(21) Application number: 22944952.5

(22) Date of filing: 22.07.2022

(51) International Patent Classification (IPC):
A61B 8/12 (2006.01)    A61B 34/20 (2016.01)
A61B 6/00 (2024.01)    A61B 6/03 (2006.01)
A61B 6/12 (2006.01)

(52) Cooperative Patent Classification (CPC):
A61B 6/00; A61B 6/03; A61B 6/12; A61B 8/12;
A61B 34/20

(86) International application number:
PCT/JP2022/028524

(87) International publication number:
WO 2023/233676 (07.12.2023 Gazette 2023/49)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 31.05.2022 JP 2022088430

(71) Applicant: ASAHI INTECC CO., LTD.
Seto-shi, Aichi, 489-0071 (JP)

(72) Inventors:
• SHIBAYAMA Yukari
  Seto-shi, Aichi 489-0071 (JP)
• YOSHITAKE Shumpei
  Seto-shi, Aichi 489-0071 (JP)
• ICHIKAWA Tomoki
  Seto-shi, Aichi 489-0071 (JP)

(74) Representative: Winter, Brandl - Partnerschaft
mbB
Alois-Steinecker-Straße 22
85354 Freising (DE)

(54) **SURGERY ASSISTANCE DEVICE, SURGERY ASSISTANCE METHOD, AND COMPUTER PROGRAM**

(57) A surgery assistance device includes a true lumen information acquisition unit that acquires three dimensional position information of a true lumen existing in a target blood vessel, a true lumen image generation unit that acquires an angiographic image of the target blood vessel from a flat panel detector (FPD) arranged at a first imaging position and generates a true lumen image representing the true lumen at a position and posture corresponding to the angiographic image by using position information of the first imaging position and three dimensional position information of the true lumen, and an image composition unit that generates a composite image by compositing the angiographic image and the true lumen image and outputs the composite image.

Fig. 1

**Description**

TECHNICAL FIELD

The present invention relates to a technique for assisting surgery.

BACKGROUND ART

In recent years, IntraVascular UltraSound (IVUS) and a flat panel detector (FPD) have been used for imaging blood vessels for examination and treatment. The IVUS is a device that has a microminiature ultrasonic vibrator at the distal end and acquires an ultrasonic image of the inside of a blood vessel. The FPD is a device that has an X-ray tube device and an X-ray flat panel detector and acquires an X-ray image of a blood vessel. The X-ray image acquired by the FPD is also referred to as an "angiographic image". For example, Patent Literature 1 describes a device that aligns a two dimensional X-ray image of a region of interest with three dimensional ultrasonic data, determines a spatial relation between a portion of an interventional device and a target location, and displays the spatial relation. For example, Patent Literature 2 describes an X-ray diagnostic device that generates a diagram illustrating angle information of an FPD arm.

CITATION LIST

Patent Literature

[0003]

　　　Patent Literature 1: Japanese Patent No. 6770655
　　　Patent Literature 2: JP 2013-233413 A

SUMMARY OF INVENTION

TECHNICAL PROBLEM

Here, like a chronic total occlusion (CTO), the inside of a blood vessel may be obstructed by an obstruction. In this case, the obstruction in the blood vessel is removed, or a stent is placed on the side of the obstruction, thereby reopening the blood vessel. In such a procedure, it is important for an operator to correctly grasp the position of a true lumen in the blood vessel from the viewpoint of improving the accuracy of the procedure, shortening the time required for the procedure, and reducing the burden on the patient. In this regard, there is a problem that the image of the true lumen may not appear in the angiographic image due to the fact that the contrast medium does not flow into a target true lumen in the blood vessel in which the CTO has occurred. Further, even if the contrast medium flows into the target true lumen, the image of the true lumen appears on the angiographic image only for a moment when the contrast medium is passing through. Therefore, there is a problem in that it is not preferable to inject the contrast medium many times in order to confirm the position of the true lumen, which leads to an increase in burden on the patient.

In this regard, in the device described in Patent Literature 1, it is possible to navigate the interventional device to a target location (a specific point of interest), but there is a problem in that the target location cannot be determined in a case where the operator cannot grasp the position of the true lumen. In addition, there are a wide variety of techniques (approach methods including how to advance a device until reopening of a blood vessel) to be adopted in accordance with individual cases such as the state of a true lumen and the state of an obstruction. However, there is a problem in that it is difficult to apply the device described in Patent Literature 1 to such a flexible technique. Further, in the device described in Patent Literature 2, no consideration is given to the problem that the image of the true lumen may not appear in the angiographic image, and the problem that it is not preferable to inject the contrast medium many times in order to confirm the position of the true lumen.

The present invention has been made to solve at least part of the above-described problems, and an object thereof is to display an image of a true lumen of a blood vessel on an image (angiographic image) of an FPD.

SOLUTION TO PROBLEM

The present invention has been made to solve at least part of the above-mentioned problems, and can be practiced as the following forms.

　　　(1) According to an aspect of the present invention, a surgery assistance device is provided. The surgery assistance device includes a true lumen information acquisition unit that acquires three dimensional position information of a true lumen existing in a target blood vessel, a true lumen image generation unit that acquires an angiographic image of the target blood vessel from a flat panel detector (FPD) arranged at a first imaging position and generates a true lumen image representing the true lumen at a position and posture corresponding to the angiographic image by using position information of the first imaging position and three dimensional position information of the true lumen, and an image composition unit that generates a composite image by compositing the angiographic image and the true lumen image and outputs the composite image.

According to this configuration, the true lumen image generation unit can generate a true lumen image representing a true lumen at the position and posture corresponding to the angiographic image by using the position information of the first imaging position at which the angiographic image is acquired and the three dimensional position information of the true lumen acquired by the true lumen information acquisition unit. That is, the true lumen image generation unit can generate a true lumen image representing an image of the true lumen on the basis of the three dimensional position information of the true lumen even when the contrast medium does not flow into the target true lumen or when the contrast medium is not flowing. In addition, since the image composition unit generates the composite image by compositing the angiographic image at the freely-selected first imaging position and the true lumen image representing the image of the true lumen and outputs the composite image, the image of the true lumen of the blood vessel can be displayed on the image (angiographic image) of the FPD. Therefore, by checking the composite image, the operator can proceed with the procedure while checking the positional relation between the medical device on the angiographic image and the true lumen on the true lumen image. As a result, since the operator can correctly grasp the position of the true lumen in the target blood vessel, it is possible to improve the accuracy of the procedure, to shorten the time required for the procedure, and to reduce the burden on the patient.

(2) In the surgery assistance device of the above aspect, the three dimensional position information of the true lumen may include information on a width of the true lumen, and the true lumen image generation unit may generate a true lumen image representing the true lumen having a width corresponding to the three dimensional position information of the true lumen.

According to this configuration, since the true lumen image generation unit generates the true lumen image representing the true lumen having the width corresponding to the three dimensional position information of the true lumen, the operator can proceed with the procedure while checking the width of the true lumen by checking the composite image. As a result, it is further possible to improve the precision of the procedure, to shorten the time required for the procedure, and to reduce the burden on the patient.

(3) In the surgery assistance device of the above aspect, when the FPD is moved to a second imaging position different from the first imaging position and the target blood vessel is captured by the FPD, the true lumen image generation unit may reacquire an angiographic image captured at the second imaging position, and regenerate a true lumen image representing the true lumen at a position and posture corresponding to the reacquired angiographic image by using position information of the second imaging position and three dimensional position information of the true lumen, and the image composition unit may regenerate a composite image by compositing the reacquired angiographic image and the regenerated true lumen image, and output the composite image.

According to this configuration, when the FPD is moved to the second imaging position different from the first imaging position and imaging is performed by the FPD, the true lumen image generation unit regenerates the true lumen image corresponding to the angiographic image at the second imaging position, and the image composition unit regenerates the composite image by compositing the reacquired angiographic image and the regenerated true lumen image, and outputs the composite image. That is, the true lumen image generation unit and the image composition unit can follow the movement of the imaging position of the FPD and display the composite image including the true lumen image after the movement. As a result, the convenience of the surgery assistance device can be improved, and it is further possible to improve the precision of the procedure, to shorten the time required for the procedure, and to reduce the burden on the patient.

(4) The surgery assistance device of the above aspect may further include an angiographic image acquisition unit that acquires a first angiographic image captured by the FPD arranged at a first position and a second angiographic image captured by the FPD arranged at a second position different from the first position and an ultrasonic image acquisition unit that acquires an ultrasonic image of an inside of the target blood vessel captured by an ultrasonic sensor. The first angiographic image may include the ultrasonic sensor arranged at a first mark position within the target blood vessel, and a medical device different from the ultrasonic sensor arranged at a second mark position within the target blood vessel. The second angiographic image may include the ultrasonic sensor arranged at the first mark position within the target blood vessel. The ultrasonic image may be an image captured in a state where the ultrasonic sensor is arranged at the first mark position. The ultrasonic image may include the target blood vessel and the medical device arranged at the second mark position within the target blood vessel. The true lumen information acquisition unit may acquire three dimensional position information of the true lumen by using position information of the first position, the first angiographic image, position information of the second position, the second angiographic image, and the ultrasonic image.

According to this configuration, the true lumen information acquisition unit can acquire the three dimensional position information of the true lumen by using the position information of the first position at which the first angiographic image is acquired, the first angiographic image, the position information of the second position at which the second angiographic image is acquired, the second angiographic image, and the ultrasonic image. To be specific, the true lumen information acquisition unit can acquire the three dimensional position information of the ultrasonic sensor by using the position information of the first position and the first angiographic image, and the position information of the second position and the second angiographic image. Then, the true lumen information acquisition unit can acquire the three dimensional position information of the true lumen by using the three dimensional position information of the ultrasonic sensor, the position information of the first position, the first angiographic image, and the ultrasonic image in which the true lumen of the target blood vessel appears.

(5) In the surgery assistance device of the above aspect, the true lumen information acquisition unit may acquire a position of the ultrasonic sensor by using images of the ultrasonic sensor included in the first angiographic image and the second angiographic image, associate a positional relation between the first angiographic image and the ultrasonic image by using images of the medical device included in the first angiographic image and the ultrasonic image, acquire position information of the true lumen from the ultrasonic image, and acquire three dimensional position information of the true lumen by using the acquired position of the ultrasonic sensor and position information of the true lumen in the ultrasonic image by the ultrasonic sensor.

According to this configuration, the true lumen information acquisition unit can acquire the three dimensional position information of the ultrasonic sensor by using the image of the ultrasonic sensor included in the first angiographic image and the second angiographic image. Further, the true lumen information acquisition unit can associate the positional relation between the first angiographic image and the ultrasonic image by using the image of the medical device included in the first angiographic image and the ultrasonic image, acquire the position information of the true lumen from the ultrasonic image, and acquire the three dimensional position information of the true lumen by using the acquired position of the ultrasonic sensor and the position information of the true lumen in the ultrasonic image by the ultrasonic sensor.

(6) The surgery assistance device of the above aspect may further include an angiographic image acquisition unit that acquires a first angiographic image captured by the FPD arranged at a first position and a second angiographic image captured by the FPD arranged at a second position different from the first position. The first angiographic image may include the true lumen of the target blood vessel and a medical device arranged at a first mark position within the target blood vessel. The second angiographic image may include the true lumen of the target blood vessel and the medical device arranged at the first mark position within the target blood vessel. The true lumen information acquisition unit may acquire three dimensional position information of the true lumen by using an image of the medical device and an image of the true lumen, which are included in the first angiographic image and the second angiographic image.

According to this configuration, the true lumen information acquisition unit can acquire the three dimensional position information of the true lumen by using the images of the medical devices and the images of the true lumen included in the first angiographic image and the second angiographic image.

The present invention has been made to solve at least part of the above-mentioned problems, and can be practiced as the following forms. For example, the present invention can be implemented in the form of an information processing apparatus that outputs a composite image, an information processing apparatus that outputs an FPD imaging position recommended range together with a composite image, an FPD that outputs a composite image, an FPD that outputs an FPD imaging position recommended range together with a composite image, a system including these apparatuses, a computer program that implements the functions of these apparatuses and system, a server apparatus that distributes the computer program, and a non-transitory storage medium that stores the computer program.

BRIEF DESCRIPTION OF DRAWINGS

Fig. 1 is an explanatory diagram illustrating a configuration of a surgery assistance system.
Figs. 2A to 2D are diagrams illustrating an imaging position of a first FPD.
Figs. 3A and 3B are diagrams illustrating a target blood vessel and a device to be used.
Fig. 4 is a flowchart illustrating an example of composite image output processing.
Fig. 5 is a flowchart illustrating an example of the composite image output processing.
Figs. 6A and 6B are diagrams illustrating a screen used in the composite image output processing.
Figs. 7A and 7B are diagrams illustrating steps S5 and S7 of the composite image output processing.
Figs. 8A to 8E are diagrams illustrating step S8 of the composite image output processing.

## EP 4 534 020 A1

Fig. 9 is a diagram illustrating steps S12 to S17 of the composite image output processing.

Figs. 10A and 10B are diagrams illustrating step S18 of the composite image output processing.

Fig. 11 is a diagram illustrating calculation of a length of a vector.

Figs. 12A and 12B are diagrams illustrating steps S19 to S21 of the composite image output processing.

Fig. 13 is a diagram illustrating an example of an ultrasonic image displayed on a canvas after step S21 of the composite image output processing.

Fig. 14 is a diagram illustrating step S22 of the composite image output processing.

Fig. 15 is a diagram illustrating step S23 of the composite image output processing.

Fig. 16 is a diagram illustrating steps S24 to S28 of the composite image output processing.

Figs. 17A to 17C are diagrams illustrating step S30 of the composite image output processing.

Figs. 18A and 18B are diagrams illustrating step S30 of the composite image output processing.

Figs. 19A and 19B are diagrams illustrating an example of a composite image.

Figs. 20A and 20B are diagrams illustrating a modification of a true lumen image.

Fig. 21 is a diagram illustrating a modification of the true lumen image.

Fig. 22 is an explanatory diagram illustrating a configuration of a surgery assistance system of a second embodiment.

Fig. 23 is a flowchart illustrating an example of composite image output processing of the second embodiment.

Figs. 24A and 24B are diagrams illustrating the composite image output processing of the second embodiment.

Figs. 25A and 25B are diagrams illustrating step S18A of the composite image output processing of the second embodiment.

Fig. 26 is an explanatory diagram illustrating a configuration of a surgery assistance system of a third embodiment.

EMBODIMENTS OF THE INVENTION

<First Embodiment>

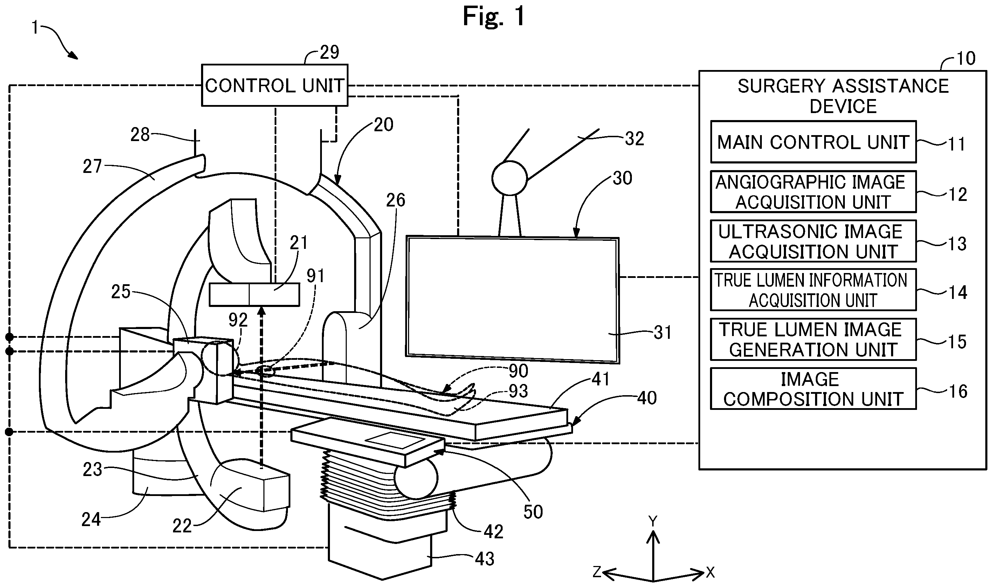

Fig. 1 is an explanatory diagram illustrating a configuration of a surgery assistance system 1. The surgery assistance system 1 is a system that supports examination and treatment. The surgery assistance system 1 includes a surgery assistance device 10, a blood vessel imaging device 20 having an FPD, a display apparatus 30, a table 40, and an operation unit 50. The surgery assistance system 1 of the present embodiment includes a surgery assistance device 10 to be described below, and thus, for a captured image (hereinafter, also referred to as an "angiographic image") of a target blood vessel captured by an FPD, can generate a true lumen image representing a true lumen at a position and posture corresponding to the angiographic image and display a composite image by compositing the angiographic image and the true lumen image. Although the term "target blood vessel" means a blood vessel to be targeted for examination or treatment, the surgery assistance system 1 may be used not only for a blood vessel system, but also for a biological lumen such as a lymph gland system, a biliary tract system, a urinary tract system, a respiratory tract system, a digestive organ system, a secretory gland, or a genital organ.

XYZ axes orthogonal to each other are illustrated in Fig. 1. The X-axis corresponds to the width direction of the blood vessel imaging device 20, the Y-axis corresponds to the height direction of the blood vessel imaging device 20, and the Z-axis corresponds to the depth direction of the blood vessel imaging device 20. In the following description, a direction in which a head 92 of a patient (Fig. 1: a human body 90) is present is also simply referred to as a "Z-axis direction" and simply represented as "Z". Three dimensional space formed by three dimensional coordinates (XYZ coordinates) formed by the X, Y, and Z axes is referred to as an XYZ three dimensional space. Note that an origin O of the XYZ three dimensional space (XYZ coordinates) is the position of a heart 91 of the human body 90.

In composite image output processing to be described below, the surgery assistance device 10 generates a true lumen image representing a true lumen at a position and posture corresponding to an angiographic image captured by the FPD, and outputs a composite image obtained by compositing the angiographic image and the true lumen image. The surgery assistance device 10 is configured to include a central processing unit (CPU), a read only memory (ROM), and a random access memory (RAM), and the CPU executes a computer program stored in the ROM, thereby implementing functions of a main control unit 11, an angiographic image acquisition unit 12, an ultrasonic image acquisition unit 13, a true lumen information acquisition unit 14, a true lumen image generation unit 15, and an image composition unit 16. The surgery assistance device 10 is electrically connected to each of a control unit 29 of the blood vessel imaging device 20, a display apparatus 30, and the operation unit 50.

The main control unit 11 transmits and receives information to and from the control unit 29 of the blood vessel imaging device 20, the display apparatus 30, and the operation unit 50, and controls the entire surgery assistance device 10. Further, the main control unit 11 controls the entire composite image output processing to be described below.

In the composite image output processing, the angiographic image acquisition unit 12 acquires a first angiographic image and a second angiographic image from the blood vessel imaging device 20. The "first angiographic image" is an angiographic image captured by the FPD arranged at a freely-selected imaging position. The imaging position of the FPD when the first image is acquired is also referred to as a "first position". The "second angiographic image" is an angiographic image captured by setting the FPD at a freely-selected imaging position different from the first position. The imaging position of the FPD when the second image is acquired is also referred to as a "second position". The details of the first and second angiographic images and the first and second positions will be described below. The process (step) executed by the angiographic image acquisition unit 12 is also referred to as an angiographic image acquisition process (step).

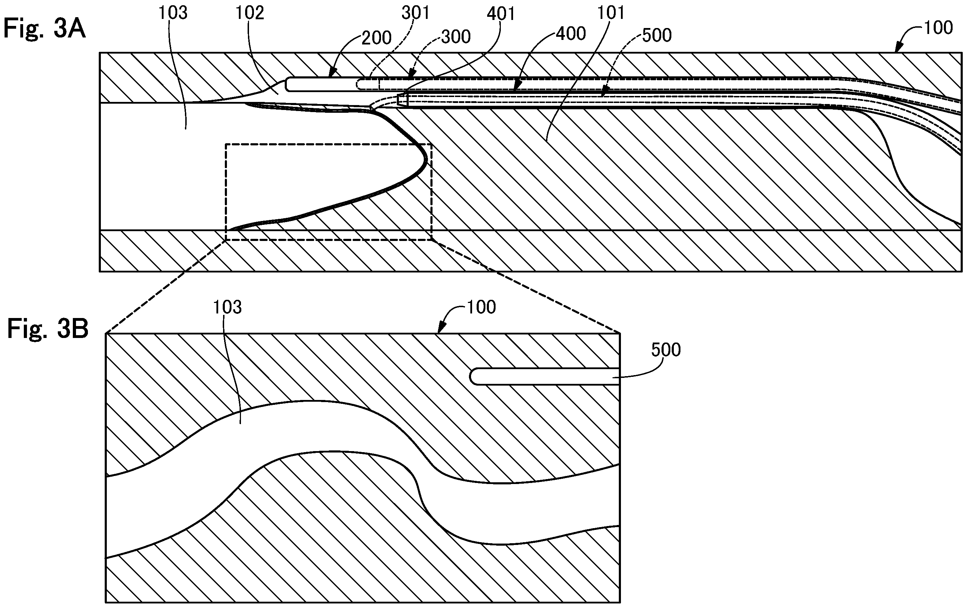

In the composite image output processing, the ultrasonic image acquisition unit 13 acquires, from the imaging sensor 300 (Figs. 3A and 3B), an ultrasonic image of the inside of the target blood vessel captured by the imaging sensor 300. Details will be described below. The process (step) executed by the ultrasonic image acquisition unit 13 is also referred to as an ultrasonic image acquisition process (step).

In the composite image output processing, the true lumen information acquisition unit 14 acquires three dimensional position information (position information in the XYZ three dimensional space) of the true lumen existing in the target blood vessel by using position information of the first position, the first angiographic image, position information of the second position, the second angiographic image, and the ultrasonic image. Details will be described below. The process (step) executed by the true lumen information acquisition unit 14 is also referred to as a true lumen information acquisition process (step).

In the composite image output processing, the true lumen image generation unit 15 generates, for the angiographic image captured by an FPD arranged at a freely-selected imaging position (hereinafter, also referred to as a "first imaging position"), a true lumen image representing a true lumen at a position and posture corresponding to the angiographic image. Further, when the FPD is moved to a freely-selected imaging position (hereinafter, also referred to as a "second imaging position") different from the first imaging position and imaging is performed by the FPD, the true lumen image generation unit 15 regenerates, for the angiographic image captured at the second imaging position, a true lumen image representing the true lumen at the position and posture corresponding to the angiographic image. Here, the first imaging position and the second imaging position mean freely-selected positions different from the first position and the second position described above, that is, freely-selected positions at which the operator intends to check the target blood vessel and the device. However, the first imaging position and the second imaging position may be the same as the first position and the second position described above. Details will be described below. The process (step) executed by the true lumen image generation unit 15 is also referred to as a true lumen image generation process (step).

In the composite image output processing, the image composition unit 16 generates a composite image by compositing the angiographic image captured by the FPD arranged at the first imaging position and the true lumen image generated by the true lumen image generation unit 15, and displays the composite image on the display apparatus 30. Further, the image composition unit 16 regenerates a composite image by compositing the angiographic image captured by the FPD arranged at the second imaging position and the true lumen image regenerated by the true lumen image generation unit 15, and displays the composite image on the display apparatus 30. Details will be described below. The process (step) executed by the image composition unit 16 is also referred to as an image composition process (step).

The blood vessel imaging device 20 has the FPD, acquires X-rays transmitted through a human body, and converts the X-rays into a digital signal to acquire an image (angiographic image). The blood vessel imaging device 20 has a first FPD 21, a first X-ray tube device 22, a first C arm 23, a first support portion 24, a second FPD 25, a second X-ray tube device 26, a second C arm 27, a second support portion 28, and the control unit 29.

The first FPD 21 includes an X-ray flat panel detector, converts X-rays entering from the first X-ray tube device 22 into an electrical signal, applies analogue/digital (A/D) conversion, and generates an X-ray image. The first X-ray tube device 22 receives supply of high-voltage power from an X-ray high-voltage apparatus (not illustrated), and irradiates an X-ray beam. As indicated by a bold dashed line in the Y-axis direction in Fig. 1, an X-ray beam irradiated from the first X-ray tube device 22 enters the first FPD 21 via the human body 90. The first C arm 23 is a C-shaped arm (support) that fixes the first FPD 21 and the first X-ray tube device 22 at positions facing each other. The first support portion 24 rotatably supports the first C arm 23. That is, the first FPD 21 and the first X-ray tube device 22 can be moved to a freely-selected imaging position around the human body 90 lying on the bed 41 in a state of being fixed to positions facing each other by the first C arm 23. Hereinafter, the first FPD 21 and the first X-ray tube device 22 fixed to the first C arm 23 is also simply referred to as "first FPD 21".

The configuration of the second FPD 25 is the same as that of the first FPD 21. The configuration of the second X-ray tube device 26 is the same as that of the first X-ray tube device 22. As indicated by a bold dashed line extending in the X-axis direction in Fig. 1, the X-ray beam irradiated from the second X-ray tube device 26 enters the second FPD 25 via the human body 90. The second C arm 27 is a C-shaped arm (support) that fixes the second FPD 25 and the second X-ray tube device 26 at positions facing each other. The second support portion 28 rotatably supports the second C arm 27. That is, the second FPD 25 and the second X-ray tube device 26 can be moved to a freely-selected imaging position around the human body 90 in a state of being fixed to positions facing each other by the second C arm 27. Hereinafter, the second FPD 25 and the second X-ray tube device 26 fixed to the second C arm 27 are simply referred to as a "second FPD 25".

The second FPD 25 is generally arranged in a direction normal to the first FPD 21. For example, as illustrated in Fig. 1, when the first FPD 21 is arranged at an imaging position in a front direction of the human body 90 (a vertical direction of the human body 90 and a longitudinal direction of the human body 90), the second FPD 25 is located at an imaging position in a horizontal direction of the human body 90 (a lateral direction of the human body 90). The blood vessel imaging device 20 may be simply referred to as an "FPD", an "FPD device", or the like.

The control unit 29 includes a CPU, a ROM, and a RAM. The CPU executes a computer program stored in the ROM to control the entire blood vessel imaging device 20. The control unit 29 is electrically connected to each of the first FPD 21, the second FPD 25, the first support portion 24, the second support portion 28, the display apparatus 30, the table 40, and the operation unit 50. The control unit 29 causes the display apparatus 30 to display the X-ray image generated by the first FPD 21 and the second FPD 25. Further, the control unit 29 drives the first support portion 24 to rotate the first C arm 23 and drives the second support portion 28 to rotate the second C arm 27 in accordance with an operation from the operation unit 50. Furthermore, in accordance with an operation from the operation unit 50, the control unit 29 changes the height of the bed 41 by expanding and contracting an expansion/contraction portion 42, and changes the position of the bed 41 by moving the table 40 in the Z-axis direction.

The display apparatus 30 is connected to the surgery assistance device 10 and the control unit 29 of the blood vessel imaging device 20, and functions as an output interface for the surgery assistance device 10 and the blood vessel imaging device 20. The display apparatus 30 includes a monitor 31 and an arm 32. The monitor 31 is a "display unit" constituted by a well-known means such as a liquid crystal display, smart glasses, or a projector. The arm 32 supports and fixes the monitor 31.

The table 40 is a table for laying the human body 90 and positioning the human body 90 near the first FPD 21 and the second FPD 25. The table 40 has the bed 41, the expansion/contraction portion 42, and a leg portion 43. The bed 41 includes a mattress on which the human body 90 is laid. The bed 41 is supported by the table 40 so as to be movable in the Z-axis direction. The expansion/contraction portion 42 is configured to be able to change the height of the bed 41 by expanding and contracting in the Y-axis direction. The leg portion 43 supports the bed 41 and the expansion/contraction portion 42. As illustrated by a broken line in Fig. 1, the human body 90 is laid on the bed 41 so as to face upward in a state where the head 92 is placed on the side close to the first FPD 21 and the second FPD 25 and feet 93 are placed on the side far from the first FPD 21 and the second FPD 25. In this way, it is easy to acquire an image of the target blood vessel in the heart 91 by the first FPD 21 and the second FPD 25.

The operation unit 50 is connected to the surgery assistance device 10 and the control unit 29 of the blood vessel imaging device 20, and functions as an input interface for the surgery assistance device 10 and the blood vessel imaging device 20. The operation unit 50 is an "input unit" constituted by well-known means such as a touch panel, an operation button, an operation lever, an operation switch, a keyboard, a mouse, a voice input unit, and a foot switch. In the illustrated example, the operation unit 50 is fixed to the table 40.

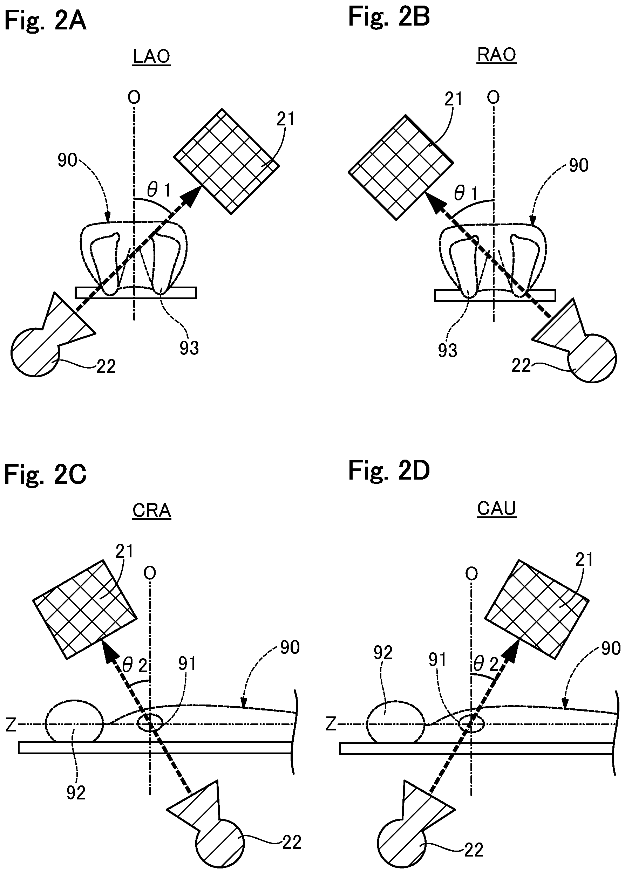

Figs. 2A to 2D are diagrams illustrating an imaging position of the first FPD 21. Fig. 2A is a diagram illustrating a left anterior oblique view (LAO), and Fig. 2B is a diagram illustrating a right anterior oblique view (RAO). As illustrated in Fig. 2A, a case where the first FPD 21 is positioned on the left side of the human body 90 is referred to as a LAO. As illustrated in Fig. 2B, a case where the first FPD 21 is positioned on the right side of the human body 90 is referred to as a RAO. Fig. 2C is a diagram illustrating cranial (CRA), and Fig. 2D is a diagram illustrating caudal (CAU). As illustrated in Fig. 2C, a case where the first FPD 21 is positioned in the upper direction of the human body 90 is referred to as CRA. As illustrated in Fig. 2D, a case where the first FPD 21 is positioned in the lower direction of the human body 90 is referred to as CAU. That is, the "imaging position of the first FPD 21" is specified by a combination of a left-right position (A1) and an up-down position (A2) described below.

(A1) LAO or RAO, and an angle θ1 from a center O of the human body 90,
(A2) CRA or CAU, and an angle θ2 from the center O of the human body 90. Here, the center O of the human body 90 is the position of the heart 91 of the human body 90 (the position of the origin O in the XYZ three dimensional space). For example, "the imaging position of the first FPD 21 is (RAO28 CRA5)" means that the first FPD 21 is at a position of 28 degrees in the right direction of the human body 90 and at a position of 5 degrees in the upper direction of the human body 90.

Figs. 3A and 3B are diagrams illustrating the target blood vessel 100 and a device to be used. Fig. 3A is a diagram illustrating a longitudinal section of the target blood vessel 100, and Fig. 3B is a diagram illustrating a part of the target blood vessel 100 surrounded by a rectangle of a broken line in Fig. 3A as viewed from above. Fig. 3A illustrates the target blood vessel 100, a CTO 101 generated in the target blood vessel 100, a false lumen 102 formed in the intima or subintima of the target blood vessel 100, and a true lumen 103. Note that the false lumen 102 means all the dissected lumens other than the true lumen 103 formed by a medical device.

Here, as illustrated in Fig. 3B, the true lumen 103 does not necessarily extend linearly, and may meander. For example, in the case of the example illustrated in Fig. 3B, in order to guide a guide wire 500 into the true lumen 103, an operator needs to direct the distal end portion of the guide wire 500 to the lower side of the plane. However, since only a fluoroscopic image in a certain cross section can be obtained by the FPD, a part of the true lumen 103 meandering as illustrated in Fig. 3A cannot be included in the angiographic image, depending on the imaging position of the FPD. In this regard, the surgery assistance device 10 of the present embodiment generates and displays a true lumen image representing the image of the true lumen by the composite image output processing to be described below, and thus it is possible to solve such a problem.

In the surgery assistance system 1, an imaging sensor 300 and the guide wire 500 illustrated in Fig. 3A are used. The imaging sensor 300 is an ultrasonic sensor that acquires an ultrasonic image of the inside of the target blood vessel 100. The imaging sensor 300 has an elongated outer shape and has a transducer 301 at a distal end portion thereof. The transducer 301 is an ultrasonic probe (also referred to as an ultrasonic vibrator, a piezoelectric body, an ultrasonic transmission/reception element, or an ultrasonic element) that transmits an ultrasonic wave toward a biological tissue and receives an ultrasonic wave propagated through and reflected by the biological tissue. The imaging sensor 300 acquires an ultrasonic image of the inside of the target blood vessel 100 around the transducer 301 while moving back and forth in the lumen of a sensor catheter 200. Specifically, the transducer 301 acquires an ultrasonic image of the inside of the target blood vessel 100 in a direction perpendicular to a transducer axis (in a 360° circumferential direction of the transducer axis) while rotating about a central axis (hereinafter, also referred to as a transducer axis) of the transducer 301 extending in the longitudinal direction of the image sensor 300.

The guide wire 500 is a medical device having an elongated outer shape. The guide wire 500 may be a plasma guide wire that includes an electrode at the distal end and performs ablation of a biological tissue with the use of a plasma flow. In this case, a wire catheter 400 may be configured to include another electrode at the distal end portion. Further, the guide wire 500 may be a penetrating guide wire that includes a pointed portion at the distal end and penetrates a biological tissue with the use of the pointed portion, or may be a delivery guide wire that does not include the pointed portion. The guide wire 500 is accommodated in the lumen of the wire catheter 400, and the distal end portion of the guide wire 500 protrudes to the outside from a distal end portion 401 of the wire catheter 400.

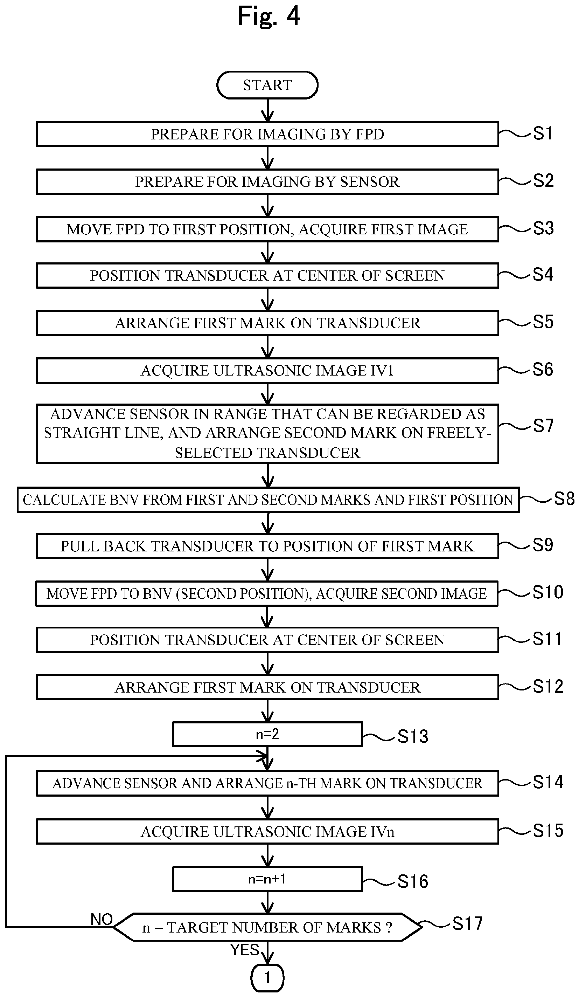

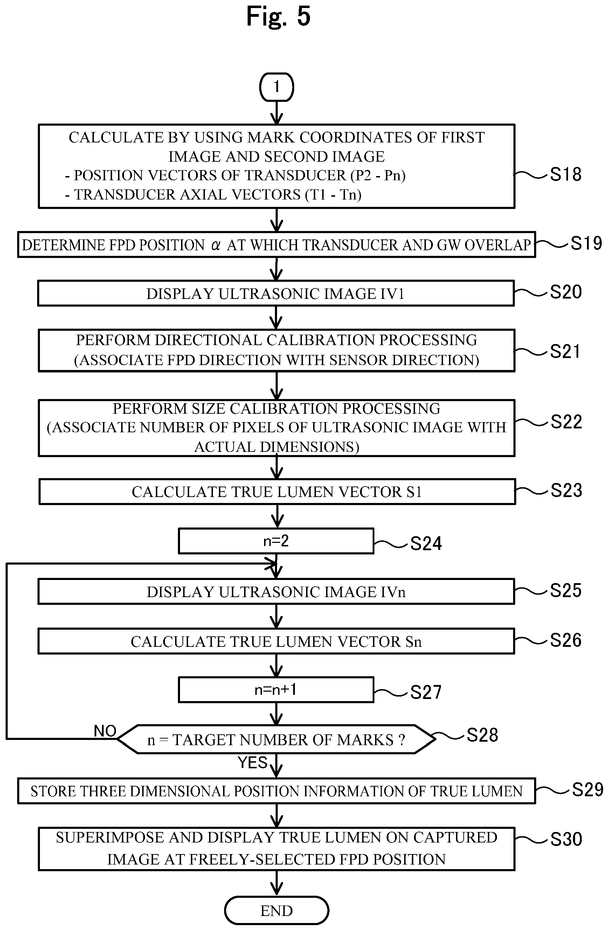

Figs. 4 and 5 are flowcharts illustrating an example of the composite image output processing. The composite image output processing can be started by a selected trigger such as power-on of the surgery assistance device 10, activation of a predetermined application, or power-on of the blood vessel imaging device 20. In the following description, a case where an angiographic image is acquired using the first FPD 21 will be exemplified. However, the angiographic image may be acquired using the second FPD 25. In this case, in the following description, the term "first FPD 21" may be replaced with the term "second FPD 25". In the flowcharts of Figs. 4 and 5, for convenience of illustration, the imaging sensor 300 is simply referred to as a "sensor", and the angiographic image is simply referred to as an "image".

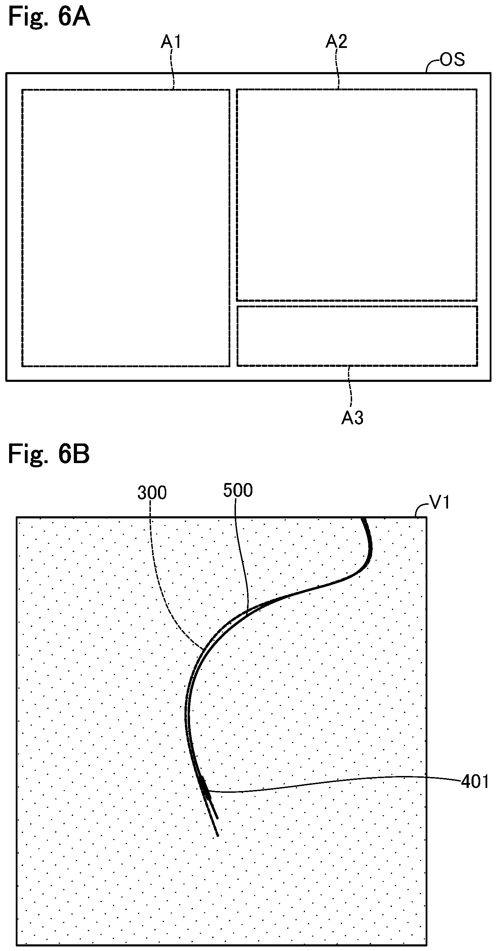

Figs. 6A and 6B are diagrams illustrating a screen used in the composite image output processing. Fig. 6A is a diagram illustrating a configuration of an operation screen OS, and Fig. 6B is a diagram illustrating an example of a first angiographic image V1. In the composite image output processing to be described below, the true lumen information acquisition unit 14 advances the processing while guiding the operation to the operator. In the example of the present embodiment, the true lumen information acquisition unit 14 causes the display apparatus 30 to display the operation screen OS such as that illustrated in Fig. 6A, and displays guidance of various operations with the use of the operation screen OS. The operation screen OS has an operation button display area A1 in which buttons for performing various operations are arranged, a canvas A2, and a guidance display area A3 in which various guidance messages are displayed. The canvas A2 is an area for displaying an angiographic image sequentially acquired by the first FPD 21 or an ultrasonic image acquired by the imaging sensor 300. In the composite image output processing, the true lumen information acquisition unit 14 performs each step while updating the angiographic image to be displayed on the canvas A2 of the operation screen OS.

The operation screen OS is merely an example, and various changes can be made. For example, the guidance display area A3 of the operation screen OS may be omitted, and voice guidance may be provided. For example, the guidance display area A3 of the operation screen OS may be omitted, and a button to which an item name is attached (for example, a button described as "arrangement of first mark" in the case of the step S5) may be arranged in the operation button display area A1 instead of guidance.

In step S1, the true lumen information acquisition unit 14 guides the operator to prepare for the imaging by the first FPD 21. In accordance with the guidance, the operator prepares for imaging by the first FPD 21. Specifically, as illustrated in Fig. 1, the human body 90 is laid on the bed 41, and the blood vessel imaging device 20 is powered on.

In step S2, the true lumen information acquisition unit 14 guides the operator to prepare for imaging by the imaging sensor 300. In accordance with the guidance, the operator prepares for imaging by the imaging sensor 300. To be specific, as illustrated in Fig. 3A, the imaging sensor 300 and the guide wire 500 are inserted into the blood vessel of the human body 90, and are delivered in such a manner that the transducer 301 of the imaging sensor 300 and the distal end portion of the guide wire 500 are positioned in the vicinity of the CTO 101 of the target blood vessel 100.

In step S3, the true lumen information acquisition unit 14 guides the operator to move the first FPD 21 to a first position and capture an X-ray image. In accordance with the guidance, the operator moves the first FPD 21 to the first position and captures an X-ray image of the target blood vessel 100 to acquire the first angiographic image V1. The first position may be a freely-selected position (RAO XX CRA XX:X is a freely-selected integer). In step S3, the true lumen information acquisition unit 14 may automatically move the first FPD 21 to the first position and perform imaging. The angiographic image acquisition unit 12 acquires the captured first angiographic image V1 from the blood vessel imaging device 20. As illustrated in Fig. 6B, the first angiographic image V1 includes an image of the imaging sensor 300, an image of the guide wire 500, and an image (not illustrated) of the target blood vessel 100 through which the imaging sensor 300 and the guide wire 500 are inserted. In Fig. 6B, for distinction, the imaging sensor 300 is indicated by a broken line, and the guide wire 500 is indicated by a solid line. In the example of the present embodiment, the first angiographic image V1 includes the image of the target blood vessel 100. However, the first angiographic image V1 may include only the image of the imaging sensor 300 and the image of the guide wire 500, and may not include the image of the target blood vessel 100. The "image of the target blood vessel 100" means an image of the contour of the target blood vessel 100.

In step S4, the true lumen information acquisition unit 14 displays the first angiographic image V1 on the canvas A2, and adjusts the position of the first angiographic image V1 in such a manner that the image of the transducer 301 (see Figs. 3A and 3B) in the image of the imaging sensor 300 is positioned at the center of the canvas A2.

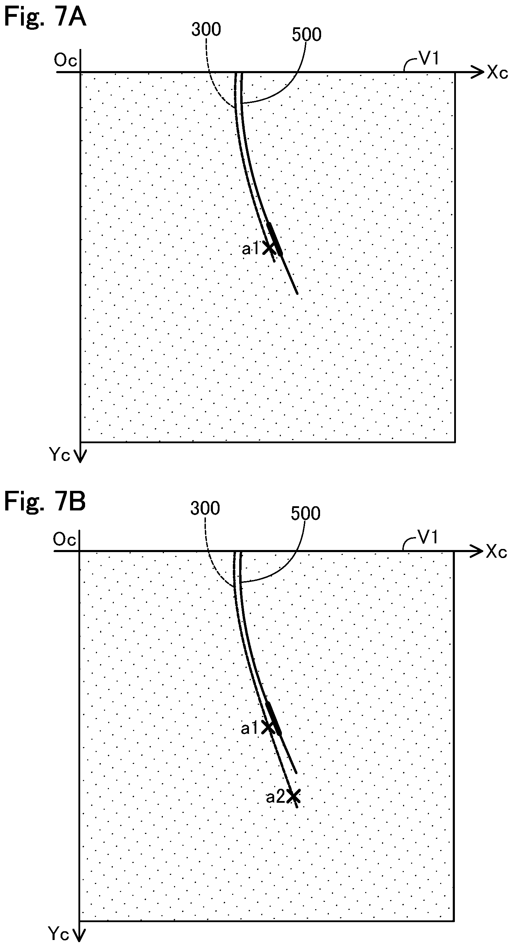

Figs. 7A and 7B are diagrams illustrating steps S5 and S7 of the composite image output processing. Fig. 7A is a diagram illustrating a state of the canvas A2 in step S5, and Fig. 7B is a diagram illustrating a state of the canvas A2 in step S7. As illustrated in Figs. 7A and 7B, the square or rectangular canvas A2 has XcYc coordinates which are two dimensional coordinates formed of an Xc-axis extending in the right direction of the plane and a Yc-axis extending in the lower direction of the plane, with a vertex at the upper left of the plane as an origin Oc. Two dimensional space formed by the XcYc coordinates is referred to as an XcYc two dimensional space. In the XcYc two dimensional space, the minus direction of the Yc-axis (the upward direction in the plane) is the direction in which the head 92 of the human body 90 (see Fig. 1) is present. In step S5, the true lumen information acquisition unit 14 guides the operator to arrange a first mark a1 on the image of the transducer 301 of the imaging sensor 300 in the first angiographic image V1 displayed on the canvas A2. In accordance with the guidance, as illustrated in Fig. 7A, the operator arranges the first mark a1 on the first angiographic image V1 on the canvas A2. The arrangement of the first mark a1 can be achieved by, for example, clicking or tapping an intended position on the image of the canvas A2. On the first angiographic image V1, when the image of the transducer 301 is located at the first mark a1 of the XcYc coordinates on the canvas A2, the actual position (position in the XYZ coordinates) of the transducer 301 in the target blood vessel 100 is represented by P1.

In step S6, the true lumen information acquisition unit 14 guides the operator to acquire an ultrasonic image IV1 with the use of the imaging sensor 300 while maintaining the position of the imaging sensor 300. In accordance with the guidance, the operator acquires the ultrasonic image IV1 from the imaging sensor 300 without moving the imaging sensor 300 from the position in Fig. 7A. The ultrasonic image acquisition unit 13 acquires the captured ultrasonic image IV1 from the imaging sensor 300, and stores the ultrasonic image IV1 in the storage unit inside the surgery assistance device 10. That is, the ultrasonic image acquisition unit 13 acquires the ultrasonic image IV1 when the transducer 301 is positioned at P1 in the target blood vessel 100.

That is, the first angiographic image V1 acquired in step S3 includes the target blood vessel 100, the image of the imaging sensor 300 arranged at the first mark position (first mark a1) in the target blood vessel 100, and the guide wire 500 arranged at the second mark position (freely-selected position different from the first mark a1) in the target blood vessel 100. The ultrasonic image IV1 acquired in step S6 includes the target blood vessel 100 and the guide wire 500 arranged at a second mark position (a freely-selected position different from the first mark a1) in the target blood vessel 100.

In step S7, the true lumen information acquisition unit 14 guides the operator to advance the imaging sensor 300 in a range that can be regarded as a straight line, and then arrange the second mark a2 on the transducer 301. Here, the "straight line" means that the trajectory of the transducer 301 when moved in the sensor catheter 200 is a straight line. In accordance with the guidance, as illustrated in Fig. 7B, the operator advances the imaging sensor 300 by a range (length) that can be regarded as a straight line, and then arranges the second mark a2 on the first angiographic image V1 on the canvas A2. When the image of the transducer 301 is located at the second mark a2 of the XcYc coordinates in the canvas A2 on the first angiographic image V1, the actual position (position in the XYZ coordinates) of the transducer 301 in the target blood vessel 100 is represented by Pe.

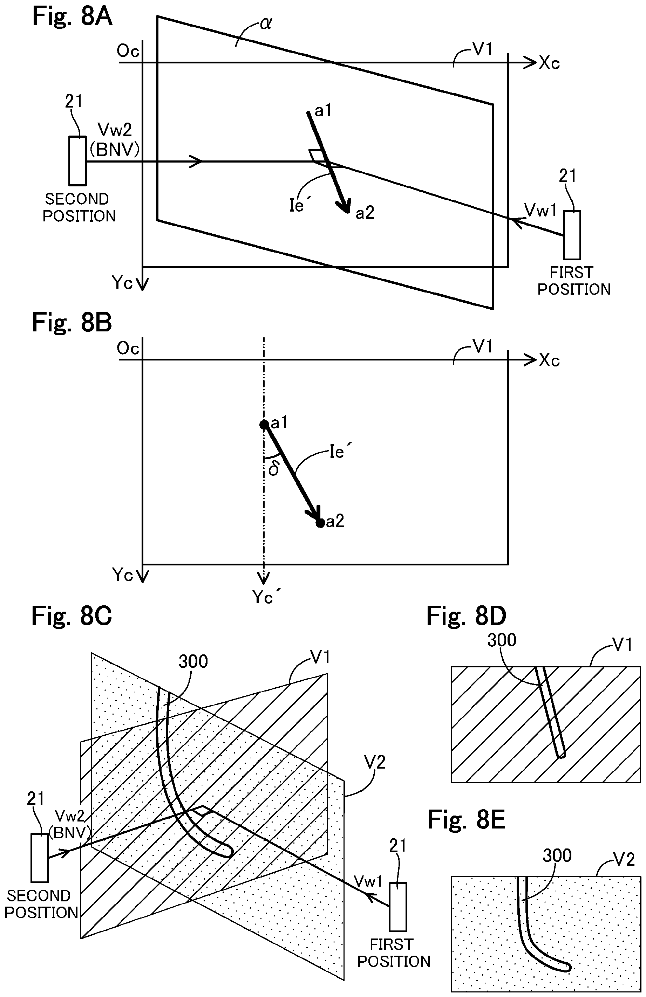

Figs. 8A to 8E are diagrams illustrating step S8 of the composite image output processing. Figs. 8A and 8B are diagrams illustrating the calculation of BNV which will be described below. Fig. 8C is a diagram illustrating a relation between the first angiographic image V1 at the first position and a second angiographic image V2 at a second position which will be described below. Fig. 8D is a diagram illustrating the first angiographic image V1 at the first position. Fig. 8E is a diagram illustrating the second angiographic image V2 at the second position.

In step S8, the true lumen information acquisition unit 14 calculates the BNV with the use of the first mark a1, the second mark a2, and the first position in the first angiographic image V1 on the canvas A2. To be more specific, as illustrated in Fig. 8A, the true lumen information acquisition unit 14 calculates the BNV with respect to a plane W including a first shaft axial vector Ie' of the imaging sensor 300, which is a vector having the first mark a1 as a start point and the second mark a2 as an end point (a vector representing a trajectory of the transducer 301 that can be regarded as a straight line), and a first view vector Vw1, which is a vector representing a first view as an imaging direction with respect to the heart 91 (see Fig. 1) in the first FPD 21 arranged at the first position. Further, since the plane W is a plane including the first shaft axial vector Ie', it can be said that the plane W is a plane on which the trajectory from the P1 (corresponding to the first mark a1) of the transducer 301 to Pe (corresponding to the second mark a2) in the XYZ three dimensional space is placed. Here, "BNV" means an imaging direction perpendicular to the plane W as illustrated in Fig. 8A. That is, BNV means an imaging direction in which the second angiographic image V2 perpendicular to the first angiographic image V1 can be acquired. The position of the first FPD 21 where the imaging direction is BNV is referred to as a "second position", and the imaging direction (= BNV) of the first FPD 21 arranged at the second position with respect to the heart 91 is referred to as a second view. A vector representing the second view is referred to as a second view vector Vw2.

The true lumen information acquisition unit 14 calculates ψ, θ, and δ by substituting a numerical value RL$_{val}$ representing LAO or RAO, a numerical value CC$_{val}$ representing CRA or CAU, and an inclination δ (see Fig. 8B) of the first shaft axial vector Ie' with respect to the Yc'-axis parallel to the Yc-axis in the canvas A2, which are the position information of the first FPD 21 at the first position (also simply referred to as "position information of the first position"), into Formula (1) indicated below. In Formula (1), "ψ" and "θ" are variables for displaying the first view vector Vw1 in polar coordinates. Further, "CW" means "clockwise", and "CCW" means "counterclockwise". Next, the true lumen information acquisition unit 14 substitutes the calculated ψ, θ, and δ into Formula (2) to calculate the orthogonal coordinates of the second view vector Vw2. Next, the true lumen information acquisition unit 14 converts the orthogonal coordinates (x, y, z) of the second view vector Vw2 into polar coordinates (r, θ, ψ) by using Formula (3). Next, the true lumen information acquisition unit 14 calculates a numerical value RL$_{val}$ representing LAO or RAO and a numerical value CC$_{val}$ representing CRA or CAU at the second position from the polar coordinates (r, θ, ψ) of the second view vector Vw2.

[Formula 1]

$$
\begin{aligned}
RAO &\rightarrow \quad \varphi = 90 - RL_{val} \\
LAO &\rightarrow \quad \varphi = 90 + RL_{val} \\
CRA &\rightarrow \quad \theta = 90 - CC_{val} \\
CAU &\rightarrow \quad \theta = 90 + CC_{val} \\
CW &\rightarrow \quad \delta = 180 - \delta \\
CCW &\rightarrow \quad \delta = \delta
\end{aligned}
\qquad \cdots (1)
$$

[Formula 2]

$$
\begin{pmatrix} x \\ y \\ z \end{pmatrix} = \begin{pmatrix} sin\varphi cos\delta + cos\varphi cos\theta sin\delta \\ sin\varphi cos\theta sin\delta - cos\varphi cos\delta \\ -sin\theta sin\delta \end{pmatrix} \qquad \cdots (2)
$$

[Formula 3]

$$
\begin{aligned}
r &= \sqrt{x^2 + y^2 + z^2} \\
\theta &= \cos^{-1}\left(\frac{z}{\sqrt{x^2 + y^2 + z^2}}\right) \qquad \cdots (3) \\
\varphi &= \tan^{-1}\left(\frac{y}{x}\right)
\end{aligned}
$$

A case where the imaging sensor 300 is in a posture as illustrated in Fig. 8C in the target blood vessel 100 will be considered. At this time, since the imaging sensor 300 is advanced in the range that can be regarded as a straight line in step S7, it can be said that the first angiographic image V1 is an image acquired from a direction in which the imaging sensor 300 can be seen as a straight line, that is, the first view at the first position. Therefore, in the first angiographic image V1, the imaging sensor 300 is captured in a linear shape as in the images with diagonal hatching in Figs. 8C and 8D. On the other hand, since the second view (BNV) at the second position is perpendicular to the first view, it can be said that the second angiographic image V2 obtained by the second view at the second position is perpendicular to the first angiographic image V1 and is an image obtained from a direction in which the imaging sensor 300 (the trajectory of the transducer 301) appears to be curved. Therefore, in the second angiographic image V2, the imaging sensor 300 is captured in a curved shape as in the images with dot hatching in Figs. 8C and 8E.

In step S9, the true lumen information acquisition unit 14 guides the operator to pull back the transducer 301 of the imaging sensor 300 to the position of the first mark a1. In accordance with the guidance, the operator pulls back the transducer 301 to the position of the first mark a1. That is, the operator pulls back the transducer 301 in the target blood vessel 100 from the position Pe to P1 in the XYZ coordinates.

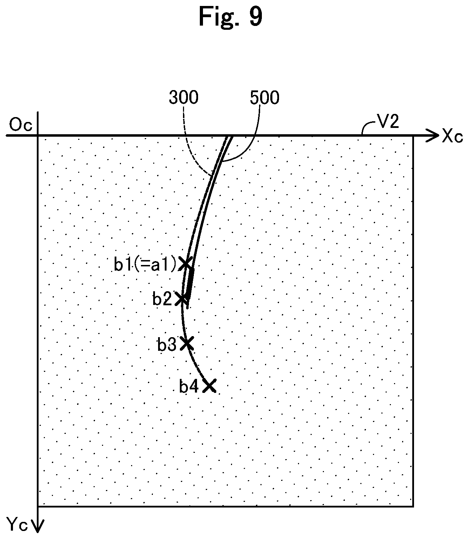

In step S10, the true lumen information acquisition unit 14 guides the operator to move the first FPD 21 to the second position corresponding to the BNV calculated in step S8, and capture an X-ray image. In accordance with the guidance, the operator moves the first FPD 21 to the second position and captures an X-ray image of the target blood vessel 100 to acquire a second angiographic image V2. In step S10, the true lumen information acquisition unit 14 may automatically move the first FPD 21 to the second position and perform imaging. The angiographic image acquisition unit 12 acquires the captured second angiographic image V2 from the blood vessel imaging device 20. As described below with reference to Fig. 9, the second angiographic image V2 includes an image of the imaging sensor 300, an image of the guide wire 500, and an image (not illustrated) of the target blood vessel 100 through which the imaging sensor 300 and the guide wire 500 are inserted, which are captured from a direction different from that of the first angiographic image V1. In the example of the present embodiment, the second angiographic image V2 includes the image of the target blood vessel 100. However, the second angiographic image V2 may include only the image of the imaging sensor 300, and may not include the image of the target blood vessel 100 or the image of the guide wire 500.

In step S11, the true lumen information acquisition unit 14 displays the second angiographic image V2 on the canvas A2, and adjusts the position of the second angiographic image V2 in such a manner that the image of the transducer 301 in the image of the imaging sensor 300 is positioned at the center of the canvas A2.

Fig. 9 is a diagram illustrating steps S12 to S17 of the composite image output processing. Fig. 9 illustrates an example of the second angiographic image V2 on the canvas A2. In step S12, the true lumen information acquisition unit 14 guides the operator to arrange a first mark b1 on the image of the transducer 301 of the imaging sensor 300 in the second angiographic image V2 displayed on the canvas A2. In accordance with the guidance, the operator arranges the first mark b1 on the second angiographic image V2 on the canvas A2. The arrangement of the first mark b1 can be achieved by, for example, clicking or tapping an intended position on the image of the canvas A2. Since the transducer 301 is pulled back to the position of the first mark a1 in step S9, the actual position of the first mark b1 in the XYZ three dimensional space is the same as the position of the first mark a1 added in step S5 (Fig. 9: b1 = a1). That is, on the second angiographic image V2, when the image of the transducer 301 is at the first mark b1 of the XcYc coordinates on the canvas A2, the actual position (position in the XYZ coordinates) of the transducer 301 in the target blood vessel 100 is P1.

In step S13, the true lumen information acquisition unit 14 substitutes 2 into a variable n used in the composite image output processing. N is a natural number. In step S14, the true lumen information acquisition unit 14 guides the operator to advance the imaging sensor 300 by a freely-selected length and arrange a second mark b2 on the transducer 301. In accordance with the guidance, the operator advances the imaging sensor 300, and then arranges the second mark b2 on the second angiographic image V2 on the canvas A2. When the image of the transducer 301 is located at the second mark b2 of the XcYc coordinates in the canvas A2 on the second angiographic image V2, the actual position (position in the XYZ coordinates) of the transducer 301 in the target blood vessel 100 is represented by P2.

In step S15, the true lumen information acquisition unit 14 guides the operator to acquire the ultrasonic image IV2 with the use of the imaging sensor 300 while maintaining the position of the imaging sensor 300. In accordance with the guidance, the operator acquires the ultrasonic image IV2 from the imaging sensor 300 without moving the imaging sensor 300. The ultrasonic image acquisition unit 13 acquires the captured ultrasonic image IV2 from the imaging sensor 300, and stores the ultrasonic image IV2 in the storage unit inside the surgery assistance device 10. That is, the ultrasonic image acquisition unit 13 acquires the ultrasonic image IV2 when the transducer 301 is positioned at P2 in the target blood vessel 100. In step S16, the true lumen information acquisition unit 14 adds 1 to the variable n.

In step S17, the true lumen information acquisition unit 14 determines whether the arrangement of the marks on the second angiographic image V2 (step S14) and the acquisition of the ultrasonic images at the positions of the marks (step S15) have been completed for the target number of marks. The target number of marks can be freely determined, and n = 4 in the case of Fig. 9. When the variable n reaches the target number of marks (step S17: YES), the true lumen information acquisition unit 14 shifts the processing to step S18. When the variable n has not reached the target number of marks (step S17: NO), the true lumen information acquisition unit 14 shifts the processing to step S14 and repeats the above-described processing. As a result, as illustrated in Fig. 9, the n-th mark bn is arranged on the second angiographic image V2 while incrementing the variable n such as n = 3 and n = 4 (step S14), and an ultrasonic image IVn corresponding to the position of each n-th mark bn is acquired (step S15). When the image of the transducer 301 is located at the n-th mark bn of the XcYc coordinates in the canvas A2 on the second angiographic image V2, the actual position (position in the XYZ coordinates) of the transducer 301 in the target blood vessel 100 is represented by Pn. Further, the range in which the imaging sensor 300 is advanced on the second angiographic image V2 is a range in which the imaging sensor 300 can be regarded as a straight line on the first angiographic image V1, that is, from the first mark a1 to the second mark a2 (from the position P1 to Pe in the target blood vessel 100).

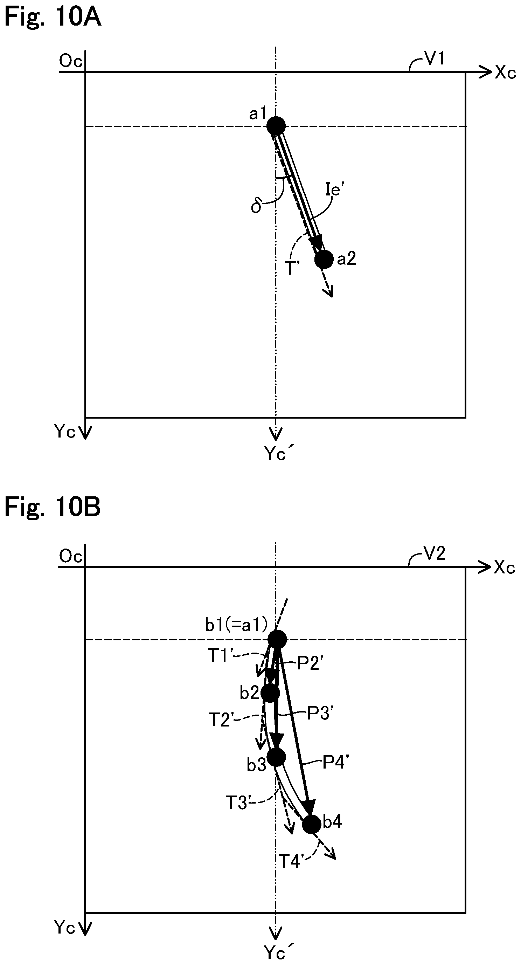

Figs. 10A and 10B are diagrams illustrating step S18 of the composite image output processing. Fig. 10A is a diagram illustrating each point on the first angiographic image V1 displayed on the canvas A2, and Fig. 10B is a diagram illustrating each point on the second angiographic image V2 displayed on the canvas A2. In Figs. 10A and 10B, the upper side of the plane is oriented toward the Z-axis of the XYZ coordinates (Fig. 1: the direction in which the head 92 of the human body 90 is present). That is, the minus direction of the Yc-axis of the canvas A2 is the direction in which the head 92 of the human body 90 in Fig. 1 is present. In Figs. 10A and 10B, the Yc'-axis is a straight line parallel to the Yc-axis.

In step S18, the true lumen information acquisition unit 14 calculates the following (B1) and (B2) with the use of each coordinate in the XcYc coordinates of the first mark a1 and the second mark a2 in the first angiographic image V1 displayed on the canvas A2, and each coordinate in the XcYc coordinates of the first mark b1 to the n-th mark bn in the second angiographic image V2.

(B1) Position vectors P2 to Pn in the XYZ three dimensional space of the transducer 301: the position vectors P2 to Pn of the transducer 301 are vectors extending from the position P1 serving as a reference point (start point) in the target blood vessel 100 to the positions P2 to Pn, respectively.

(B2) Transducer axial vectors T1 to Tn of the transducer 301 of the imaging sensor 300: the transducer axial vectors T1 to Tn are vectors of the transducer axes (the central axes of the transducer 301 extending in the longitudinal direction of the imaging sensor 300) when the transducer 301 is positioned at the positions P1 to Pn in the target blood vessel 100. Here, it can be said that the transducer axial vector when the transducer 301 is positioned at the positions P1 to Pn is a tangent vector at the positions P1 to Pn on the trajectory of the transducer 301.

Here, as described with reference to Figs. 8A to 8E, since the first angiographic image V1 and the second angiographic image V2 displayed on the canvas A2 are images obtained by capturing the imaging sensor 300 from different angles, it can be said that the first mark b1 to the n-th mark bn (n = 4 in the illustrated example) in the second angiographic image V2 illustrated Fig. 10B are on the first shaft axial vector Ie' of the image sensor 300 in the first angiographic image V1 illustrated in Fig. 10A. Therefore, the direction of the "(B1) position vectors P2 to Pn of the transducer 301" can be calculated using the inclination $\delta$ of the first shaft axial vector Ie' with respect to the Yc-axis (see Fig. 10A) and the inclination of the second shaft axial vector P2' to Pn' of the transducer 301 on the second angiographic image V2 with respect to the Yc-axis. The first shaft axial vector Ie' is a vector extending from the first mark a1 to the second mark a2 on the first angiographic image V1. Further, the second shaft axial vector P2' is a vector extending from the first mark b1 to the second mark b2 on the second angiographic image V2, and the second shaft axial vector Pn' is a vector extending from the first mark b1 to the n-th mark bn.

Further, in the first angiographic image V1 illustrated in Fig. 10A, it can be said that the tangent vector T' in the trajectory of the transducer 301 passing through the first mark a1 and the second mark a2 is on the first shaft axial vector Ie'. Therefore, the direction of the "(B2) transducer axial vectors T1 to Tn of the transducer 301 of the imaging sensor 300" can be calculated using the inclination $\delta$ of the shaft axial vector Ie' in the first angiographic image V1 with respect to the Yc-axis and the inclination of the tangent vectors T1' to Tn' in the second angiographic image V2 with respect to the Yc-axis. The tangent vector T1' in the second angiographic image V2 is a tangent vector at the first mark b1 on the trajectory of the transducer 301 extending from the first mark b1 to the n-th mark bn. Similarly, a tangent vector Tn' is a tangent vector at the n-th mark bn on the trajectory of the transducer 301.

The details of a method for obtaining the direction of a vector of an object positioned on a straight line where a plane defined by two vectors, i.e., a first view vector Vw1 representing the imaging direction of the first FPD 21 at the first position and the first shaft axial vector Ie' representing the trajectory of the transducer 301 appearing on the first angiographic image V1 and a plane defined by a second view vector Vw2 representing the imaging direction of the first FPD 21 at the second position and the second shaft axial vectors Pn' to Pn' of the transducer 301 appearing on the second angiographic image V2 intersect are disclosed in International Application PCT/JP2021/034980. In the International Application PCT/JP2021/034980, by using a blood vessel existing plane H2 viewed from the first position and a blood vessel existing plane S viewed from the second position, a straight line where the H2 surface and the S surface intersect is defined, and a "blood vessel axial vector" which is the straight line is calculated. In the example of the present embodiment, for example, the plane defined by two vectors, i.e., the first view vector Vw1 representing the imaging direction of the first FPD 21 at the first position and the first shaft axial vector Ie' representing the trajectory of the transducer 301 appearing on the first angiographic image V1 corresponds to the plane H2, and the plane defined by the second view vector Vw2 representing the imaging direction of the first FPD 21 at the second position and the second shaft axial vectors P2' to Pn' appearing on the second angiographic image V2 corresponds to the plane S, and the position vectors P2 to Pn calculated in the above-described (B1) may be calculated as corresponding to the blood vessel axial vector. Further, the transducer axial vectors T1 to Tn calculated in the above (B2) can be similarly calculated as a vector corresponding to the blood vessel axial vector.

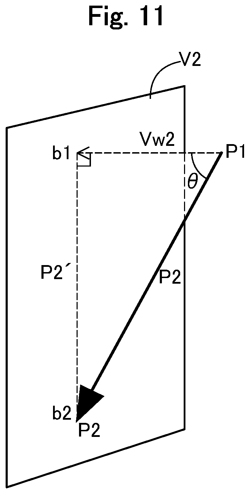

Fig. 11 is a diagram illustrating the calculation of the lengths of the position vectors P2 to Pn of the transducer 301. To be specific, Fig. 11 is a diagram illustrating, as an example, the calculation of the length of the position vector P2 of the transducer 301 when the transducer 301 moves from the start point (reference point) P1 to the end point P2 in the target blood vessel 100. The length of the position vector Pn can be calculated in the same manner. In Fig. 11, the vector Vw2 is the second view vector Vw2 (see Fig. 8A) representing the imaging direction when the first FPD 21 is at the second position. Further, b1 is the first mark b1 on the second angiographic image V2, and is the position of the transducer 301 on the second angiographic image V2 when the transducer 301 is positioned at the start point P1. Similarly, b2 is the second mark b2 in the second angiographic image V2, and is the position of the transducer 301 on the second angiographic image V2 when the transducer 301 is positioned at the end point P2. The vector P2' is the second shaft axial vector P2' of the transducer 301 on the second angiographic image V2, and is an orthogonal projection vector of the position vector P2 of the transducer 301 onto the second angiographic image V2. Further, $\theta$ is an angle formed by the second view vector Vw2 and the position vector P2. In step S18, the true lumen information acquisition unit 14 calculates the lengths of the above-described "(B) position vectors P2 to Pn of the transducer 301" by using the following Formula (4). When the second view vector Vw2 and the position vector P2 are unit vectors, $\theta$ can be calculated by the first formula of Formula (4) based on the formula of the inner product of the vectors. Further, the length of the second shaft axial vectors P2' can be calculated from the coordinates of b1 and b2 in the XcYc coordinates of the canvas A2, and hence the length of the position vector P2 can be derived by the second formula of the Formula (4). In the first formula of Formula (4), "Vw2 × P2" represents the inner product of the second view vector Vw2 and the position vector P2, and in the second formula of Formula (4), "P2" and "P2'" represent the length of the position vector P2 and the second shaft axial vector P2', respectively.

[Formula 4]

$$\theta = \cos^{-1}(\text{Vw2} \times \text{P2})$$
$$\text{P2} = \text{P2'} / \sin\theta \qquad \cdots (4)$$

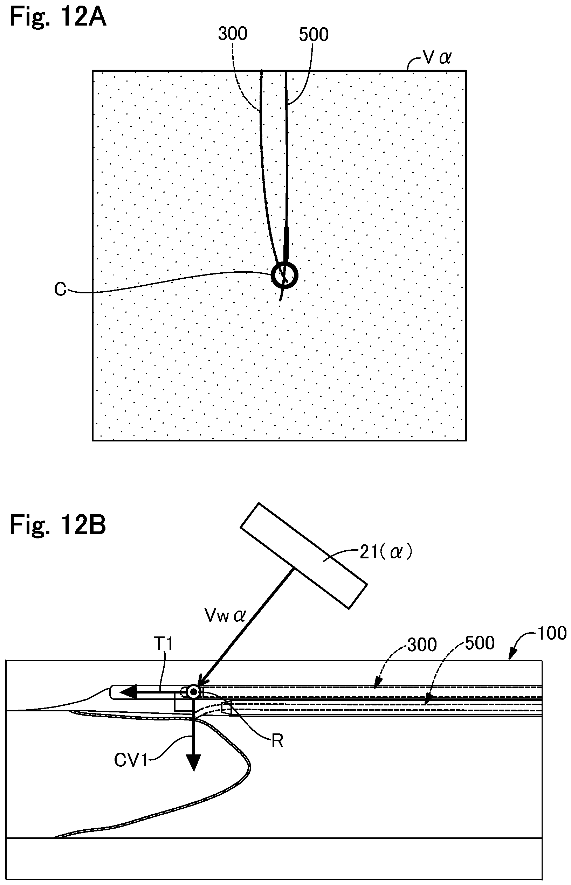

Figs. 12A and 12B are diagrams illustrating steps S19 to S21 of the composite image output processing. Fig. 12A is a diagram illustrating an example of an angiographic image V$\alpha$ when the first FPD 21 is arranged at the position $\alpha$. Fig. 12B is a diagram illustrating a relation among the first FPD 21, the imaging sensor 300, and the guide wire 500 when the first FPD 21 is arranged at the position $\alpha$.

In step S19, the true lumen information acquisition unit 14 guides the operator to pull back the transducer 301 of the imaging sensor 300 to the position of the first mark b1 (= a1). In accordance with the guidance, the operator pulls the transducer 301 back to the position of the first mark b1. Further, the true lumen information acquisition unit 14 guides the operator to search for the position $\alpha$ of the first FPD 21 at which the angiographic image V$\alpha$ in which the transducer 301 of the imaging sensor 300 overlaps with the guide wire 500 (in other words, the transducer 301 of the imaging sensor 300 intersects with the guide wire 500) is obtained. In accordance with the guidance, the operator moves the first FPD 21 to the position $\alpha$ at which the imaging sensor 300 and the guide wire 500 can be seen in an overlapping manner as illustrated in Fig. 12A. That is, the angiographic image V$\alpha$ is an angiographic image captured by the first FPD 21 arranged at the position $\alpha$ where the transducer 301 and the guide wire 500 can be seen in an overlapping manner when the transducer 301 is positioned at P1 in the target blood vessel 100.

In step S20, the true lumen information acquisition unit 14 displays the ultrasonic image IV1 (the ultrasonic image when the transducer 301 is positioned at the first mark b1 on the second angiographic image V2, that is, the ultrasonic image when the transducer 301 is positioned at P1 in the target blood vessel 100) on the canvas A2.

In step S21, the true lumen information acquisition unit 14 performs directional calibration processing (processing of associating the direction from the transducer 301 toward the guide wire 500 in the XYZ three dimensional space with the direction from the transducer 301 toward the guide wire 500 in the ultrasonic image IV1 displayed in the XcYc two dimensional space of the canvas A2). To be specific, the true lumen information acquisition unit 14 acquires the position $\alpha$ of the first FPD 21 in the step S19. Here, as illustrated in Fig. 12B, a vector representing the imaging direction with respect to the heart 91 (see Fig. 1) of the first FPD 21 arranged at the position $\alpha$ is defined as a view vector Vw$\alpha$. The true lumen information acquisition unit 14 calculates the view vector Vw$\alpha$ from the acquired position $\alpha$ of the first FPD 21. The true lumen information acquisition unit 14 calculates a rotation axis R (r1, r2, r3) with the use of the outer product of the transducer axial vector T1 of the imaging sensor 300 at the position P1 in the target blood vessel 100 calculated in the above (B2) and the view vector Vw$\alpha$, as indicated in Formula (5).

[Formula 5]

$$R = \text{T1} \times \text{Vw}\alpha$$
$$(R = (r1, r2, r3)) \qquad \cdots (5)$$

Thereafter, as illustrated in Formula (6), the true lumen information acquisition unit 14 calculates a vector CV1 obtained by rotating the transducer axial vector T1 of the transducer 301 calculated in the above (B2) by 90 degrees about the rotation axis R obtained by Formula (5). That is, the vector CV1 is a vector that extends from the transducer 301 toward the guide wire 500 when the transducer 301 is at the position P1 in the XYZ three dimensional space and is perpendicular to the transducer axial vector T1. Formula (7) is a matrix representation of Rodrigues' rotation formula indicated in Formula (6).

[Formula 6]

EP 4 534 020 A1

$$CV1 = Rn(90)T1 \qquad \cdots (6)$$

[Formula 7]

$$Rn(\theta) = \begin{pmatrix} r1^2(1-\cos\theta)+\cos\theta & r1r2(1-\cos\theta)-r3\sin\theta & r1r3(1-\cos\theta)+r2\sin\theta \\ r1r2(1-\cos\theta)+r3\sin\theta & r2^2(1-\cos\theta)+\cos\theta & r2r3(1-\cos\theta)-r1\sin\theta \\ r1r3(1-\cos\theta)-r2\sin\theta & r2r3(1-\cos\theta)+r1\sin\theta & r3^2(1-\cos\theta)+\cos\theta \end{pmatrix} \qquad \cdots (7)$$

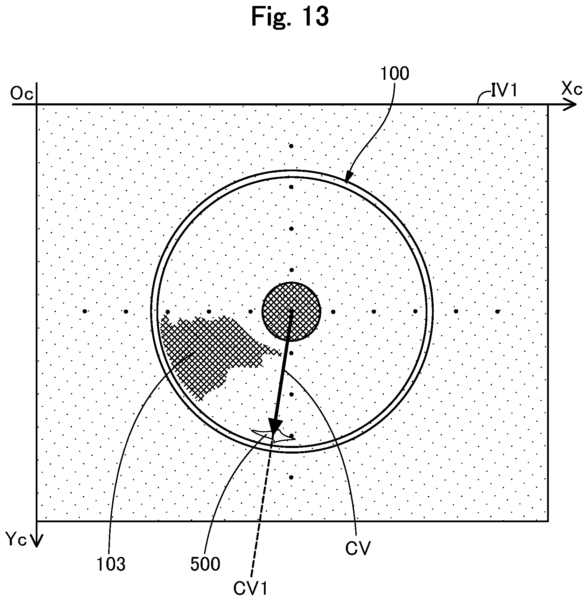

Fig. 13 is a diagram illustrating an example of the ultrasonic image IV1 displayed on the canvas A2 in step S20. In Fig. 13, the ultrasonic image IV1 is an ultrasonic image of the inside of the target blood vessel 100 in a direction perpendicular to the transducer axis T1 (in all circumferential directions of 360° of the transducer axis T1) when the transducer 301 is located at the first mark b1 of the second angiographic image V2. As illustrated in Fig. 13, the ultrasonic image IV1 includes an image of the guide wire 500 (a portion appearing relatively white as compared with the surroundings), an image 103 of the true lumen (a portion appearing relatively black as compared with the surroundings), and an image of a portion where the transducer 301 is positioned (a portion located near the center of the ultrasonic image IV1 and appearing relatively black as compared with the surroundings). Further, in Fig. 13, an arrow CV directed from the center of the ultrasonic image IV1 (that is, the center of the transducer 301) to the center of the image of the guide wire 500 is indicated. The extending direction of the arrow CV in the XcYc two dimensional space of the canvas A2 corresponds to the direction of the calculated vector CV1 in the XYZ three dimensional space. Thus, in step S21, the direction from the transducer 301 to the guide wire 500 in the XYZ three dimensional space is associated with the direction from the transducer 301 to the guide wire 500 in the ultrasonic image IV1 displayed in the XcYc two dimensional space of the canvas A2.

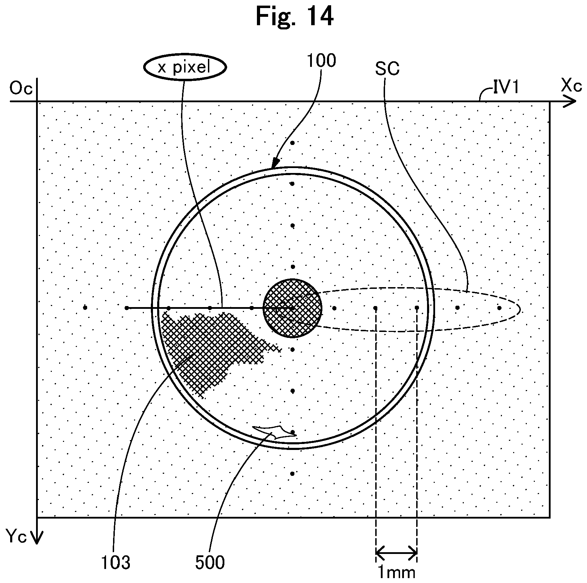

Fig. 14 is a diagram illustrating step S22 of the composite image output processing. In step S22, the true lumen information acquisition unit 14 performs size calibration processing (processing of associating the number of pixels of the ultrasonic image IV1 displayed on the canvas A2 with the actual dimensions of the ultrasonic image IV1). As illustrated in Fig. 14, the ultrasonic image IV1 is provided with a scale SC representing the actual dimensions of the target blood vessel 100. In the present embodiment, the interval between adjacent scales is 1 mm. The true lumen information acquisition unit 14 draws a line segment from the center of the image of the target blood vessel 100 to a scale outside the contour of the image of the target blood vessel 100, and measures the number of pixels of the line segment on the ultrasonic image IV1 of the canvas A2 (Fig. 14: x pixel). The true lumen information acquisition unit 14 calculates the number of pixels on the canvas A2 per 1mm of actual dimensions by calculating the number of measured pixels/the length of the line segment.

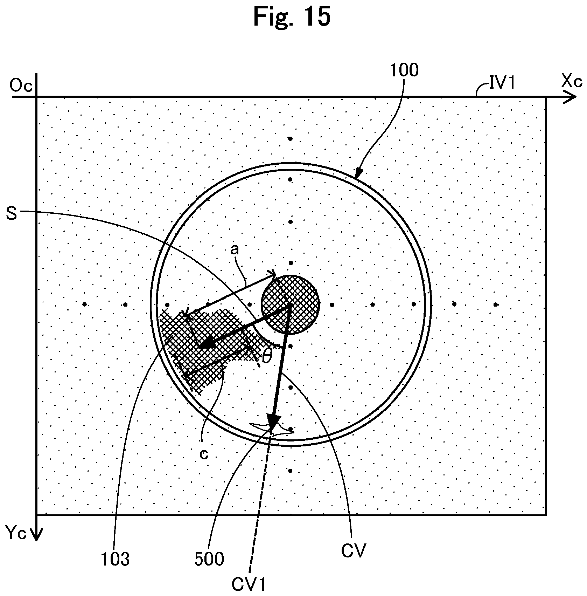

Fig. 15 is a diagram illustrating step S23 of the composite image output processing. Fig. 15 illustrates an example of the ultrasonic image IV1 displayed on the canvas A2 in step S23. In step S23, the true lumen information acquisition unit 14 calculates a true lumen vector S1 (a vector which is perpendicular to the transducer axis T1 and extends from the transducer 301 to the true lumen 103) in the XYZ three dimensional space. To be specific, the true lumen information acquisition unit 14 guides the operator to draw the arrow CV from the center of the ultrasonic image IV1 (that is, the center of the transducer 301 of the imaging sensor 300) toward the center of the image of the guide wire 500 in the ultrasonic image IV1. As illustrated in Fig. 15, the image of the portion where the transducer 301 is positioned is positioned in the vicinity of the center of the ultrasonic image IV1, and appears relatively dark as compared with the surroundings. Further, since the image of the guide wire 103 appears relatively white as compared with the surroundings, the operator who views the ultrasonic image IV1 can grasp the positions of the transducer 301 and the guide wire 500. In accordance with the guidance, the operator draws the arrow CV from the center of the ultrasonic image IV1 toward the center of the image of the guide wire 500. The drawing of the arrow CV can be achieved by, for example, an operation of clicking or tapping the center of the transducer 301 and the center of the image of the guide wire 500 on the ultrasonic image IV1 of the canvas A2. A vector indicated by the arrow CV, that is, a vector extending from the center of the transducer 301 to the center of the guide wire 500 in the XcYc coordinates, is referred to as a vector cv. The true lumen information acquisition unit 14 guides the operator to draw an arrow S from the center of the ultrasonic image IV1 (that is, the center of the image of the transducer 301 of the imaging sensor 300) toward the center of the image of the true lumen 103 in the ultrasonic image IV1. As illustrated in Fig. 15, since the true lumen 103 appears relatively black as compared with the surroundings on the ultrasonic image IV1, the operator who views the ultrasonic image IV1 can grasp the position of the true lumen 103. In accordance with the guidance, the operator draws the arrow S from the center of the ultrasonic image IV1 toward the center of the image of the true lumen 103. The drawing of the arrow S can be achieved by, for example, an operation of clicking or tapping the center of the transducer 301 and the center of the image of the true lumen 103 on the image of the canvas A2. A vector indicated by the arrow S, that is, a vector extending from the center of the transducer 301 to the center of the true lumen 103 in the XcYc coordinates, is referred to as a vector s. An angle formed by the vector cv and the vector s is denoted by $\theta$.

Thereafter, the true lumen information acquisition unit 14 calculates the angle $\theta$ formed by the vector cv and the vector s from the vector cv and the vector s in the XcYc coordinates of the canvas A2 by the formula of an inner product of the vectors. Then, as indicated in Formula (8), the true lumen information acquisition unit 14 calculates the direction of the true lumen vector S1 in the XYZ three dimensional space by rotating the vector CV1 calculated in step S21 by θ degrees about the transducer axial vector T1 (r1, r2, r3) of the imaging sensor 300 calculated in the above (B2) as a rotation axis. Formula (9) is a matrix representation of Rodrigues' rotation formula indicated in Formula (8).

[Formula 8]

$$S1 = Rn(\theta)CV1$$
$$T1 = (r1, r2, r3) \qquad \cdots (8)$$

[Formula 9]

$$Rn(\theta) = \begin{pmatrix} r1^2(1-\cos\theta)+\cos\theta & r1r2(1-\cos\theta)-r3\sin\theta & r1r3(1-\cos\theta)+r2\sin\theta \\ r1r2(1-\cos\theta)+r3\sin\theta & r2^2(1-\cos\theta)+\cos\theta & r2r3(1-\cos\theta)-r1\sin\theta \\ r1r3(1-\cos\theta)-r2\sin\theta & r2r3(1-\cos\theta)+r1\sin\theta & r3^2(1-\cos\theta)+\cos\theta \end{pmatrix} \quad \cdots (9)$$

Further, the true lumen information acquisition unit 14 acquires a number of pixels a of the arrow S drawn on the XcYc coordinates of the canvas A2 and a number of pixels c corresponding to the width of the image of the true lumen 103 in the ultrasonic image IV1. The number of pixels c of the image of the true lumen 103 may be automatically acquired by analyzing the ultrasonic image IV1, or the width may be specified by the operator. Thereafter, the true lumen information acquisition unit 14 substitutes the acquired number of pixels a and a result b of the step S22 (the number of pixels b on the canvas A2 per 1mm of actual size) into Formula (10) to calculate an actual length $S_{length}$ (mm) of the true lumen vector S1. Further, the true lumen information acquisition unit 14 substitutes the acquired number of pixels c and the result b of the step S22 into Formula (11) to calculate the actual width $S_{width}$ (mm) of the true lumen of the portion corresponding to the true lumen vector S1.

[Formula 10]

$$S_{length} = \frac{a\,[pixel]}{b\,[pixel/mm]} \qquad \cdots (10)$$

[Formula 11]

$$S_{width} = \frac{c\,[pixel]}{b\,[pixel/mm]} \qquad \cdots (11)$$

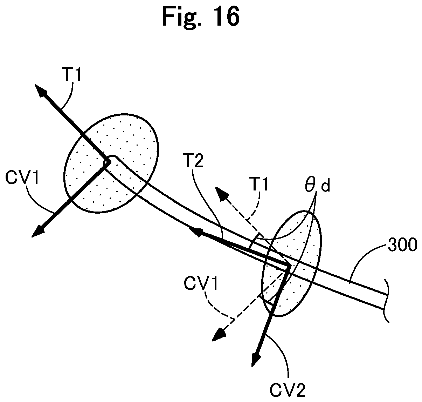

Fig. 16 is a diagram illustrating steps S24 to S28 of the composite image output processing. In step S24, the true lumen information acquisition unit 14 substitutes 2 into the variable n used in the composite image output processing. In step S25, the true lumen information acquisition unit 14 displays the ultrasonic image IV2 stored in the storage unit (that is, the ultrasonic image acquired when n = 2 in step S15) on the canvas A2.

In step S26, the true lumen information acquisition unit 14 calculates a true lumen vector Sn. To be specific, the true lumen information acquisition unit 14 guides the operator to draw an arrow S from the center of the ultrasonic image IV2 displayed on the canvas A2 toward the image of the true lumen 103 in the ultrasonic image IV2. Thereafter, the true lumen information acquisition unit 14 calculates an angle θd (Fig. 16) formed by the transducer axial vector T1 of the imaging sensor 300 calculated in the above (B2) and a transducer axial vector T2 of the imaging sensor 300. The true lumen information acquisition unit 14 calculates a vector CV2 by rotating the vector CV1 calculated in step S21 by the same angle as the calculated angle θd. The rotation axis for rotating the vector CV1 by θd is calculated by the outer product of the transducer axial vectors T1 and T2. The subsequent steps are the same as in step S23. To be specific, the true lumen information acquisition unit 14 acquires an angle θ formed by the arrow CV and the arrow S drawn in the ultrasonic image IV2. The true lumen information acquisition unit 14 calculates the direction of the true lumen vector S2 by rotating the vector CV2 by θ degrees with the transducer axial vector T2 of the imaging sensor 300 as a rotation axis as indicated in Formula (8). Further, the true lumen information acquisition unit 14 acquires the number of pixels a of the arrow S drawn in the ultrasonic image IV2 and the number of pixels c corresponding to the width of the image of the true lumen 103 in the ultrasonic image IV2. The true lumen information acquisition unit 14 calculates the actual length $S_{length}$ (mm) of the true lumen vector S2 and the actual width $S_{width}$ (mm) of the true lumen corresponding to the true lumen vector S2 by substituting the number of pixels a and the number of pixels c into Formula (10) and Formula (11), respectively. The true lumen information acquisition unit 14 can calculate a vector CVn with the use of a difference θd between the transducer axial vector T1 and the transducer axial vector Tn in the same manner for n = 3 and thereafter. In step S27, the true lumen information acquisition unit 14 adds 1 to the variable n.

In step S28, the true lumen information acquisition unit 14 determines whether the calculation of the true lumen vector Sn (step S26) has been completed for the target number of marks defined in steps S14 to S17. When the variable n reaches the target number of marks (step S28: YES), the true lumen information acquisition unit 14 shifts the processing to step S29. When the variable n has not reached the target number of marks (step S28: NO), the true lumen information acquisition unit 14 shifts the processing to step S25 and repeats the above-described processing. As a result, the ultrasonic image IVn is displayed on the canvas A2 while incrementing the variable n such as n = 3 and n = 4 (step S25), and the true lumen vector Sn is calculated (step S26).

In step S29, the true lumen information acquisition unit 14 stores the three dimensional position information (position information in the XYZ three dimensional space) of the true lumen 103 acquired in steps S23 to S28 in the storage unit inside the surgery assistance device 10. That is, in the example of the present embodiment, the three dimensional position information of the true lumen 103 includes the directions of the true lumen vectors S1 to Sn in the XYZ three dimensional space, the lengths $S_{length}$ (mm) of the true lumen vectors S1 to Sn, and the actual dimension $S_{width}$ (mm) of the true lumens of the portions corresponding to the true lumen vectors S1 to Sn. Among these, the actual dimension $S_{width}$ of the true lumen corresponds to "information on the width of the true lumen". The three dimensional position information of the true lumen may include the number of pixels c of the image of the true lumen of the portion corresponding to the true lumen vectors S1 to Sn, instead of the actual dimension $S_{width}$ of the true lumen. In this case, the number of pixels c corresponds to the "information on the width of the true lumen". In this way, even if the image of the true lumen 103 is not included in the first angiographic image V1 or the second angiographic image V2, the true lumen information acquisition unit 14 can acquire the three dimensional position information of the true lumen 103 on the basis of the information of the true lumen 103 acquired from the ultrasonic image IV1.

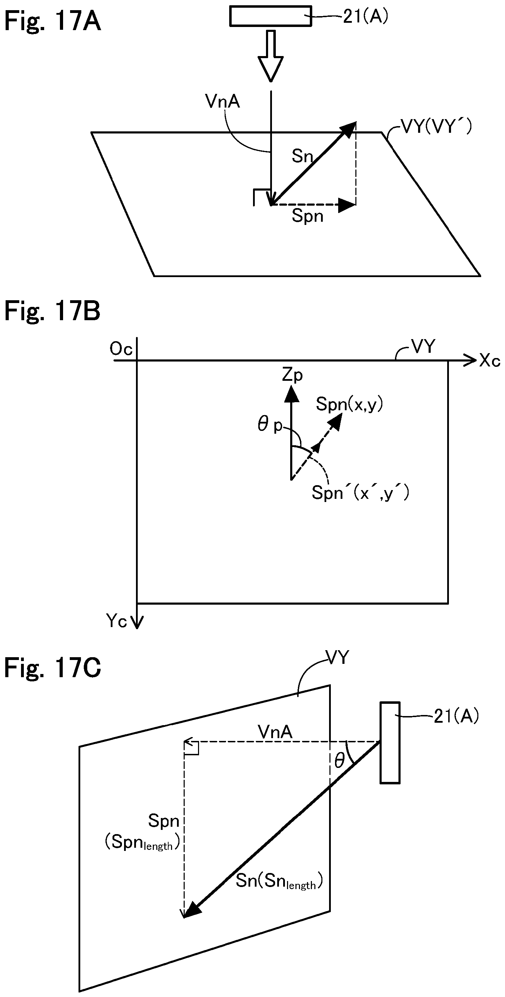

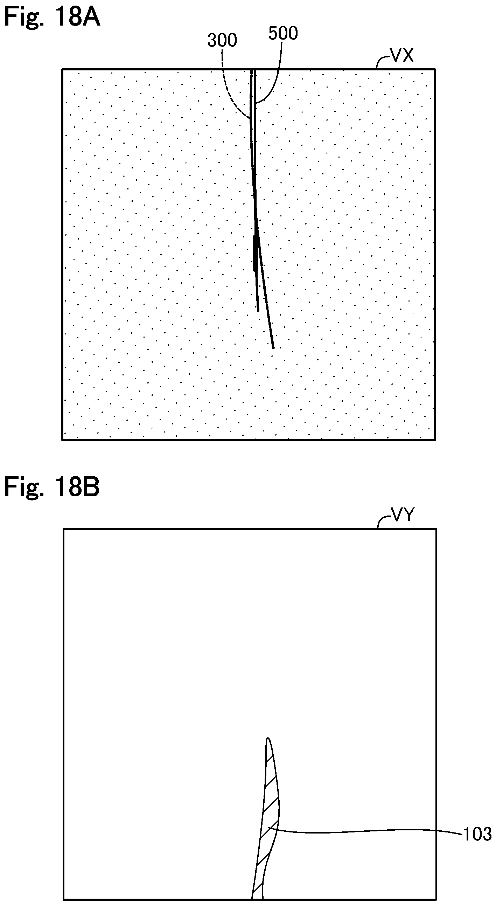

Figs. 17A to 17C and 18A and 18B are diagrams illustrating step S30 of the composite image output processing. Fig. 17A is a diagram illustrating the true lumen vector Sn in the XYZ three dimensional space, the true lumen image VY obtained by projecting (orthogonally projecting) the true lumen vector Sn onto a projection plane VY' from the imaging direction of the first FPD 21 arranged at the imaging position A (the direction of the white arrow from the upper side to the lower side of the plane), and an orthogonal projection vector Spn of the true lumen vector Sn on the true lumen image VY. Figs. 17B and 17C are diagrams illustrating the calculation of the orthogonal projection vector Spn of the true lumen vector Sn. Fig. 18A is a diagram illustrating an example of an angiographic image VX at a freely-selected FPD position. Fig. 18B is a diagram illustrating an example of a true lumen image VY corresponding to the angiographic image VX.

In step S30, the image composition unit 16 superimposes and displays a true lumen image on a captured image (angiographic image) at a freely-selected FPD position. To be specific, the true lumen image generation unit 15 and the image composition unit 16 perform processing described in the following (C1) to (C4).

(C1) The true lumen image generation unit 15 acquires the angiographic image VX obtained by imaging the target blood vessel 100 with the first FPD 21 (see Fig. 17A) arranged at a freely-selected imaging position A. The imaging position A corresponds to the "first imaging position".

(C2) The true lumen image generation unit 15 acquires the position information of the imaging position A from the first FPD 21. Further, the true lumen image generation unit 15 acquires the three dimensional position information of the true lumen from the storage unit of the surgery assistance device 10 (see Fig. 1).

(C3) The true lumen image generation unit 15 generates the true lumen image VY representing the true lumen at a position and posture corresponding to the angiographic image VX at the imaging position A by using the position information of the imaging position A and the three dimensional position information of the true lumen. A method of generating the true lumen image VY will be described below in (D1) to (D7).

(C4) The image composition unit 16 generates a composite image V by compositing the angiographic image VX and the true lumen image VY, and displays the composite image V on the canvas A2.

As illustrated in Figs. 18A and 18B, the angiographic image VX includes an image of the imaging sensor 300 viewed from a freely-selected imaging position A, an image of the guide wire 500, and an image (not illustrated) of the target blood vessel 100. Further, the true lumen image VY includes an image of the true lumen 103 at a position and posture corresponding to the angiographic image VX (in other words, when viewed from the imaging position A at which the angiographic image VX is acquired). The position of the true lumen is synonymous with the coordinates of the image of the true lumen 103 on the true lumen image VY. The posture of the true lumen is synonymous with the orientation of the image of the true lumen 103 on the true lumen image VY.

The true lumen image generation unit 15 generates the true lumen image VY by the procedure indicated in the following (D1) to (D7).

(D1) As illustrated in Fig. 17A, a vector extending perpendicularly from the imaging position A to the projection plane VY' (hereinafter, the true lumen image VY will be described as the projection plane VY') is set as a view vector VnA (that is, the view vector VnA is a vector representing an imaging direction with respect to the heart 91 (see Fig. 1) of the first FPD 21 arranged at the imaging position A). The true lumen image generation unit 15, for example, defines the view vector VnA in the XYZ three dimensional space by the first formula of Formula (12), and calculates, from the first formula of Formula (12), the second formula (the vector a1 and the vector a2) of Formula (12) representing the true lumen image VY which is a plane orthogonal to the view vector VnA.

[Formula 12]

$$VnA = (x, y, z)$$
$$a1 = (0, -z, y), \quad a2 = (y, -x, 0) \qquad \cdots (12)$$

(D2) The true lumen image generation unit 15 sets the vector a1 calculated in the procedure (D1) and the true lumen image VY defined by the vector a2 as a matrix A, and calculates, by Formula (13), a projection matrix P for calculating the orthogonal projection vector SPn to the true lumen image VY of the true lumen vector Sn included in the three dimensional position information of the true lumen 103. In Formula (13), a matrix AT means a transposed matrix of the matrix A.

[Formula 13]

$$P = A(A^T A)^{-1} A^T \qquad \cdots (13)$$

(D3) The true lumen image generation unit 15 calculates orthogonal projection vectors Spn and Zp to the true lumen image VY by Formula (14) with respect to the true lumen vector Sn and the Z-axis in the XYZ three dimensional space (Fig. 1: the direction in which the head 92 of the human body 90 is present). In Formula (14), Ze means a unit vector representing the Z-axis in the XYZ three dimensional space. Further, the orthogonal projection vectors Spn and Zp calculated by Formula (14) are vectors on the true lumen image VY represented by the XYZ coordinates.

[Formula 14]

$$Spn = PSn$$
$$Zp = PZe \qquad \cdots (14)$$
$$(Ze = (0, 0, 1))$$

(D4) The true lumen image generation unit 15 converts the orthogonal projection vector Spn calculated in the procedure (D3) into two dimensional coordinates on the true lumen image VY, and calculates the direction of the orthogonal projection vector Spn on the true lumen image VY. Specifically, first, as illustrated in Fig. 17B, the true lumen image generation unit 15 calculates an angle θp formed by the orthogonal projection vector Spn of the true lumen vector Sn onto the true lumen image VY and the orthogonal projection vector Zp of the Ze vector representing the Z-axis onto the true lumen image VY. Since the orthogonal projection vector Spn and the orthogonal projection vector Zp are calculated by Formula (14), for example, θp is calculated by the formula of the inner product of the vectors. Here, it is assumed that the orthogonal projection vector Spn and the orthogonal projection vector Zp are displayed on the canvas A2 having XcYc coordinates (that is, as illustrated in Fig. 17B, it is assumed that the true lumen image VY is displayed on the canvas A2). Then, the vector Zp can be said to be in the same direction as the Z-axis in the XYZ three dimensional space, and thus is parallel to the Yc-axis. However, since the direction of the vector Zp is the same as that of the Z-axis (vector Ze), i.e., the direction in which the head 92 of the human body 90 is present, that is, the direction from the lower side to the upper side of the plane in Fig. 17B, the direction of the vector Zp is opposite to that of the Yc-axis. Next, the true lumen image generation unit 15 sets a unit vector of the orthogonal projection vector Spn in the XcYc coordinates as an orthogonal projection unit vector Spn' (x', y'), and calculates same by Formula (15) or Formula (16). It can be said that the orthogonal projection unit vector Spn' (x', y') represents the direction of the orthogonal projection vector Spn on the true lumen image VY. Formula (15) is a case where the orthogonal projection unit vector Spn' is in a clockwise direction (CW) when viewed from the orthogonal projection vector Zp, and Formula (16) is a case where the orthogonal projection unit vector Spn' is in a counterclockwise direction (CCW) when viewed from the orthogonal projection vector Zp.

[Formula 15]

$$x' = \sin(\theta p), y' = -\cos(\theta p) \qquad \cdots (15)$$

[Formula 16]

$$x' = -\sin(\theta p), y' = -\cos(\theta p) \qquad \cdots (16)$$

(D5) The true lumen image generation unit 15 calculates an angle formed by the true lumen vector Sn and the view vector VnA in order to calculate the length of the orthogonal projection vector Spn of the true lumen vector Sn. Specifically, as illustrated in Fig. 17C, the angle formed by the true lumen vector Sn and the view vector VnA is set as $\theta$, and $\sin \theta$ is calculated by Formula (17) with the use of the true lumen vector Sn and the view vector VnA on the basis of the formula of the outer product of the vectors. In Formula (17), $VnA \times Sn$ means the outer product of the view vector VnA and the true lumen vector Sn.
[Formula 17]

$$sin\theta = \frac{|VnA \times Sn|}{|VnA||Sn|} \qquad \cdots (17)$$

(D6) When the length of the true lumen vector Sn included in the three dimensional position information of the true lumen is $Sn_{length}$ (mm), the true lumen image generation unit 15 calculates $Spn_{length}$ of the orthogonal projection vector Spn of the true lumen vector Sn by Formula (18) with the use of the $\sin \theta$ calculated in the procedure (D5) (see Fig. 17C). Next, the true lumen image generation unit 15 calculates the orthogonal projection vector Spn (x, y) of the true lumen vector Sn in the XcYc coordinates of the canvas A2 by Formula (15) or Formula (16), Formula (18), and Formula (19). It can be said that the orthogonal projection vector Spn (x, y) represents the direction and the length of the orthogonal projection vector Spn on the true lumen image VY.
[Formula 18]

$$Spn_{length} = Sn_{length} \times sin\theta \qquad \cdots (18)$$

[Formula 19]

$$x = x' \times Spn_{length}$$
$$y = y' \times Spn_{length} \qquad \cdots (19)$$

(D7) The true lumen image generation unit 15 generates the true lumen image VY by using the orthogonal projection vector Spn (x, y) obtained in the procedure (D6).

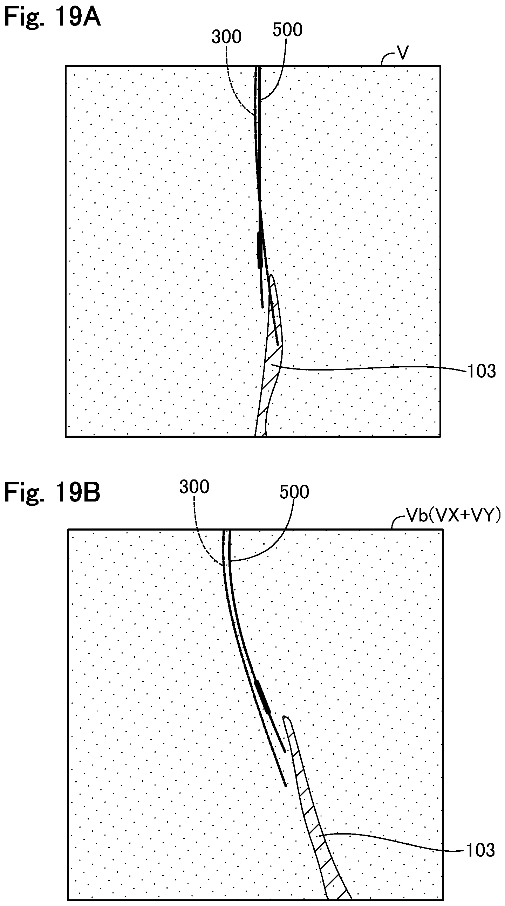

Figs. 19A and 19B are diagrams illustrating an example of a composite image. Fig. 19A is a diagram illustrating an example of the composite image V at the freely-selected imaging position A. Fig. 19B is a diagram illustrating an example of a composite image Vb at an imaging position B different from the imaging position A. By performing the processing described in the procedures (C1) to (C4), the composite image V illustrated in Fig. 19A is displayed on the canvas A2. The composite image V is an image in which the true lumen image VY is superimposed on the angiographic image VX illustrated in Fig. 18A and 18B. Here, in the true lumen image VY of the present embodiment, the image of the true lumen 103 has a position and posture corresponding to the angiographic image VX at the imaging position A, and further has a width corresponding to the three dimensional position information of the true lumen. When such a true lumen image VY is generated, the true lumen image generation unit 15 may further change the width of the image of the true lumen 103 with the use of the actual dimension $S_{width}$ (mm) of the true lumen included in the three dimensional position information of the true lumen in the procedure (D7).
Here, a case where the operator moves the first FPD 21 from the imaging position A to the imaging position B different from A will be described. The imaging position B corresponds to the "second imaging position". In such a case, the true lumen image generation unit 15 and the image composition unit 16 repeatedly execute the above-described procedures (C1) to (C4) with the use of the position information of the changed imaging position B (that is, the position information of the second imaging position). As a result, as illustrated in Fig. 19B, the composite image Vb obtained by compositing the angiographic image VX including the images of the imaging sensor 300, the guide wire 500, and the target blood vessel 100 viewed from different angles and the true lumen image VY including the image of the true lumen 103 corresponding to the angles is displayed again on the canvas A2. In the example of Fig. 19B, the image of the true lumen 103 included in the true lumen image VY is a polygon image having a width corresponding to the actual dimension $S_{width}$ (mm) of the true lumen. In this way, the operator can intuitively grasp the position, posture, and thickness of the true lumen 103, and the positional relation between the medical device (the imaging sensor 300 or the guide wire 500) and the true lumen 103.

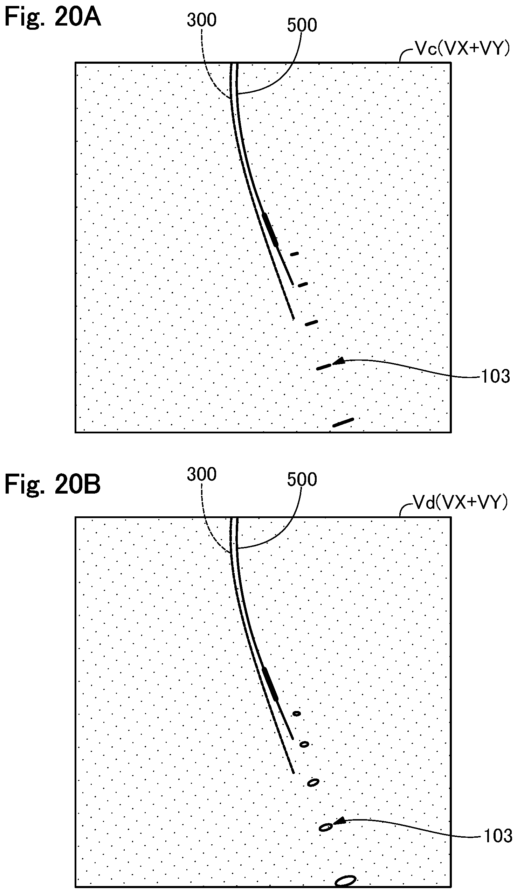

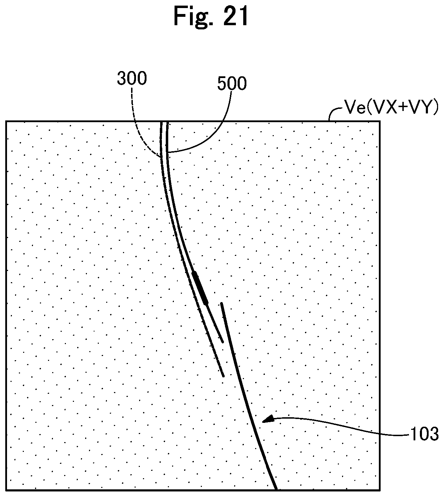

Figs. 20A and 20B and 21 are diagrams illustrating modifications of the true lumen image VY. Fig. 20A is a diagram illustrating a composite image Vc using the true lumen image VY of a first modification. Fig. 20B is a diagram illustrating a composite image Vd using the true lumen image VY of a second modification. Fig. 21 is a diagram illustrating a composite image Ve using the true lumen image VY of a third modification. In the example of Fig. 20A, the image of the true lumen 103 included in the true lumen image VY is a set of line segments having a width corresponding to the actual dimension $S_{width}$ (mm) of the true lumen. In this way, it is possible to suppress a decrease in visibility of the distal end portion of the medical device (the imaging sensor 300 or the guide wire 500) due to the image of the true lumen 103. In the example of Fig. 20A, the image of the true lumen 103 included in the true lumen image VY is a set of true lumen cross sections having a diameter corresponding to the actual dimension $S_{width}$ (mm) of the true lumen. In this way, it is possible to suppress a decrease in visibility of the distal end portion of the medical device (the imaging sensor 300 or the guide wire 500) due to the image of the true lumen 103, and it is possible to easily grasp the inclination of the true lumen 103 by the inclination of the true lumen cross section. In the example of Fig. 21, the image of the true lumen 103 included in the true lumen image VY has a position and posture corresponding to the angiographic image VX at the imaging position A, but is a line segment having no width. In this way, it is possible to reduce the processing load in the true lumen image generation unit 15.

As described above, according to the surgery assistance device 10 of the first embodiment, the true lumen image generation unit 15 can generate the true lumen image VY representing the true lumen at the position and posture corresponding to the angiographic image VX by using the position information of the first imaging position A at which the angiographic image VX is acquired and the three dimensional position information of the true lumen acquired by the true lumen information acquisition unit 14. That is, the true lumen image generation unit 15 can generate the true lumen image VY representing the image of the true lumen 103 on the basis of the three dimensional position information of the true lumen even when the contrast medium does not flow into the target true lumen 103 or when the contrast medium is not flowing. In addition, since the image composition unit 16 generates the composite image V by compositing the angiographic image VX at the freely-selected first imaging position A and the true lumen image VY representing the image of the true lumen 103 and outputs the composite image V, the image of the true lumen 103 of the target blood vessel 100 can be displayed on the image (angiographic image VX) of the FPD. Therefore, by checking the composite image V, the operator can proceed with the procedure while checking the positional relation between the medical devices 300 and 500 on the angiographic image VX and the true lumen 103 on the true lumen image VY. As a result, since the operator can correctly grasp the position of the true lumen 103 in the target blood vessel 100, it is possible to improve the accuracy of the procedure, to shorten the time required for the procedure, and to reduce the burden on the patient.

In addition, according to the surgery assistance device 10 of the first embodiment, since the true lumen image generation unit 15 generates the true lumen image VY representing the true lumen 103 having the width corresponding to the three dimensional position information of the true lumen, the operator can proceed with the procedure while checking the width of the true lumen 103 by checking the composite image V. As a result, it is further possible to improve the precision of the procedure, to shorten the time required for the procedure, and to reduce the burden on the patient.

Further, according to the surgery assistance device 10 of the first embodiment, when the first FPD 21 is moved to the second imaging position B different from the first imaging position A and imaging is performed by the first FPD 21, the true lumen image generation unit 15 regenerates the true lumen image VY corresponding to the angiographic image VX at the second imaging position B, and the image composition unit 16 regenerates the composite image Vb by compositing the reacquired angiographic image VX and the regenerated true lumen image VY, and outputs the composite image Vb. That is, the true lumen image generation unit 15 and the image composition unit 16 can follow the movement of the imaging position of the first FPD 21 and display the composite image Vb including the true lumen image VY after the movement. As a result, the convenience of the surgery assistance device 10 can be improved, and it is further possible to improve the precision of the procedure, to shorten the time required for the procedure, and to reduce the burden on the patient.

Further, according to the surgery assistance device 10 of the first embodiment, the true lumen information acquisition unit 14 can acquire the three dimensional position information of the true lumen by using the position information of the first position at which the first angiographic image is acquired, the first angiographic image, the position information of the second position at which the second angiographic image is acquired, the second angiographic image, and the ultrasonic image (Figs. 4 and 5: steps S3 to S29). To be specific, the true lumen information acquisition unit 14 can acquire the three dimensional position information of the imaging sensor 300 by using the position information of the first position and the first angiographic image V1, and the position information of the second position and the second angiographic image V2 (Fig. 5: step S18). Then, the true lumen information acquisition unit 14 can acquire the three dimensional position information of the true lumen by using the three dimensional position information of the imaging sensor 300, the position information of the first position and the first angiographic image V1, and the ultrasonic image IVn in which the true lumen 103 of the target blood vessel 100 appears (Fig. 5: steps S23 and S26).

Further, according to the surgery assistance device 10 of the first embodiment, the true lumen information acquisition unit 14 can acquire the three dimensional position information of the ultrasonic sensor by using the images of the imaging sensor 300 included in the first angiographic image V1 and the second angiographic image V2 (Fig. 5: step S18). Further, the true lumen information acquisition unit 14 can associate the positional relation between the first angiographic image V1 and the ultrasonic image IV1 by using the images of the medical device (specifically, the guide wire 500) included in the first angiographic image V1 and the ultrasonic image IV1 (Fig. 5: step S21), acquire position information of the true lumen 103 from the ultrasonic image IVn (Fig. 5: steps S23 and S26), and acquire three dimensional position information of the true lumen by using the acquired position of the imaging sensor 300 and the position information of the true lumen 103 in the ultrasonic image IVn obtained by the imaging sensor 300 (step S29).

<Second Embodiment>

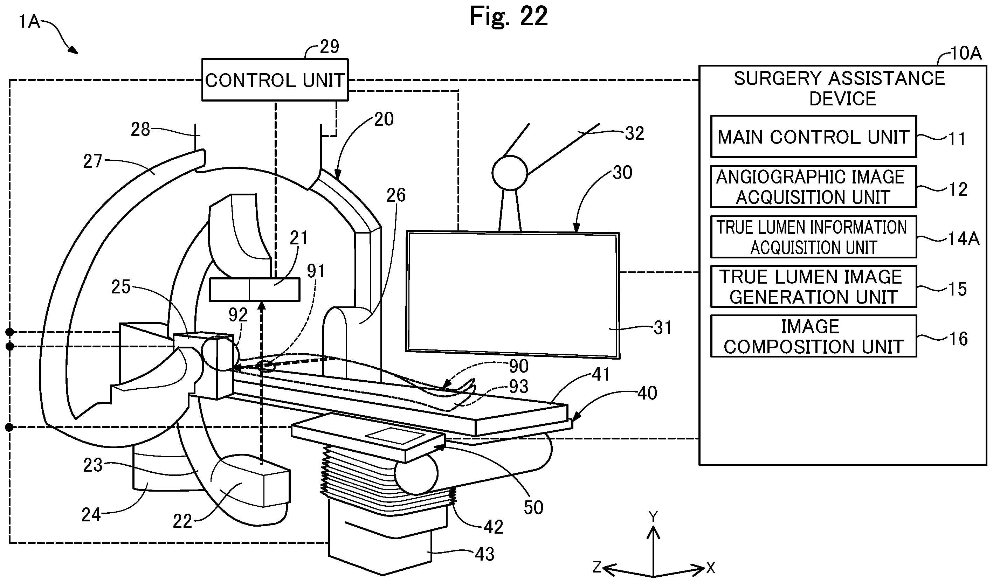

Fig. 22 is an explanatory diagram illustrating a configuration of a surgery assistance system 1A of a second embodiment. The surgery assistance system 1A of the second embodiment is different from the first embodiment in that the three dimensional position information of the true lumen is acquired without using the imaging sensor 300. The surgery assistance system 1A includes a surgery assistance device 10A instead of the surgery assistance device 10 in the configuration of the first embodiment. The surgery assistance device 10A does not include the ultrasonic image acquisition unit 13 and includes a true lumen information acquisition unit 14A instead of the true lumen information acquisition unit 14.

Since the imaging sensor 300 is not used in the surgery assistance system 1A, the wire catheter 400 and the guide wire 500 are inserted into the target blood vessel 100 described with reference to Fig. 3, but the sensor catheter 200 and the imaging sensor 300 are not inserted. In the composite image output processing, the true lumen information acquisition unit 14A acquires the three dimensional position information of the true lumen by using the image of the guide wire 500 and the image of the true lumen 103 included in the first angiographic image and the second angiographic image.

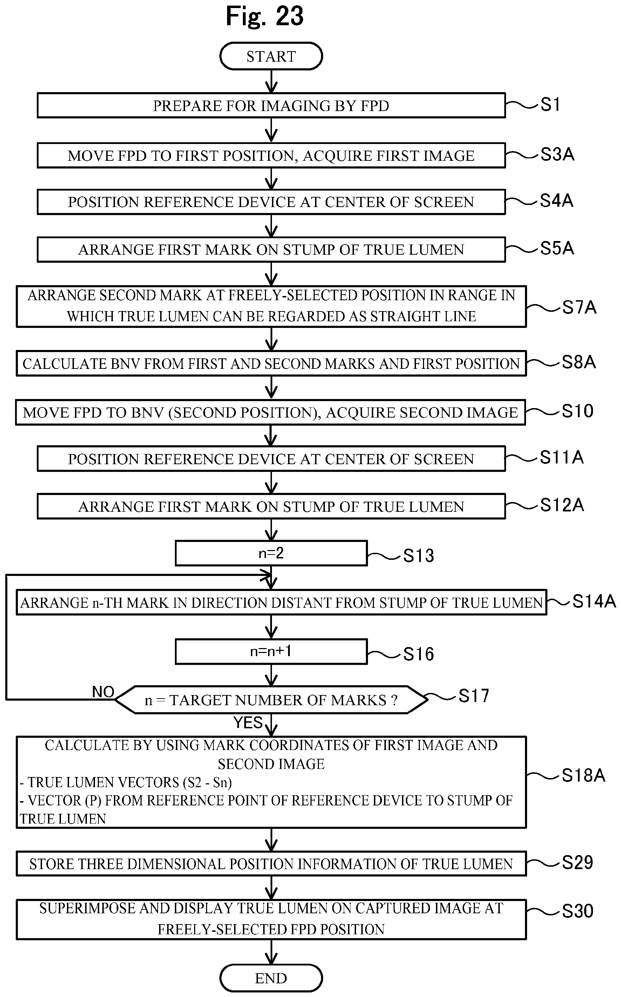

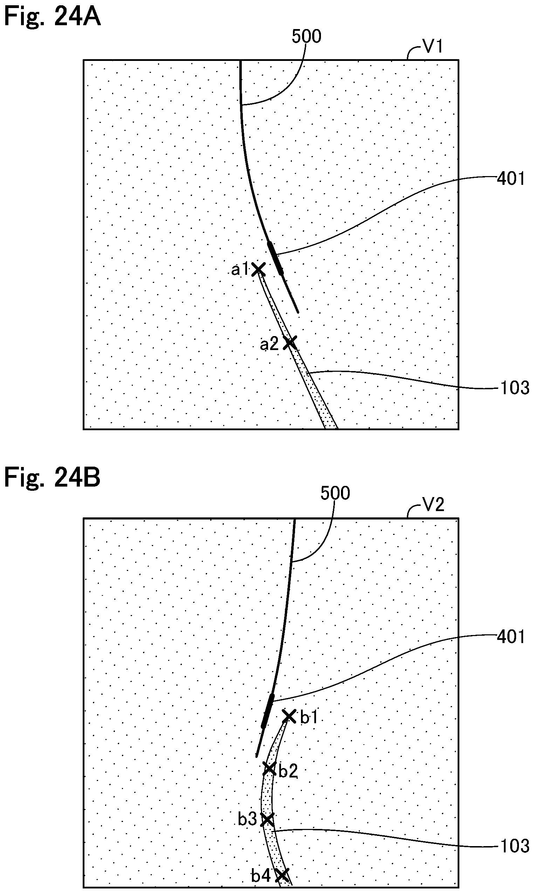

Fig. 23 is a flowchart illustrating an example of composite image output processing of the second embodiment. Figs. 24A and 24B are diagrams illustrating the composite image output processing of the second embodiment. Fig. 24A is a diagram illustrating steps S3A to S7A. Fig. 24B is a diagram illustrating steps S10 to S17. In step S1 of Fig. 23, the true lumen information acquisition unit 14A guides the operator to prepare for the imaging by the first FPD 21. The details are the same as those in step S1 of Fig. 4.

In step S3A, the true lumen information acquisition unit 14A guides the operator to move the first FPD 21 to the first position and capture an X-ray image. In accordance with the guidance, the operator moves the first FPD 21 to the first position and captures the X-ray image to acquire the first angiographic image V1. The first position may be a freely-selected position (RAOXX CRAXX:X is a freely-selected integer). The angiographic image acquisition unit 12 acquires the captured first angiographic image V1 from the blood vessel imaging device 20. As illustrated in Fig. 24A, the first angiographic image V1 includes an image of the distal end portion 401 of the wire catheter 400, an image of the guide wire 500 inserted into the wire catheter 400, an image (not illustrated) of the target blood vessel 100, and an image of the true lumen 103.

In step S4A, the true lumen information acquisition unit 14A displays the first angiographic image V1 on the canvas A2, and adjusts the position of the first angiographic image V1 in such a manner that the image of the distal end portion 401 of the wire catheter 400 is positioned at the center of the canvas A2. In the second embodiment, the processing proceeds with the distal end portion 401 of the wire catheter 400 as the "reference point of a reference device".

In step S5A, the true lumen information acquisition unit 14A guides the operator to arrange the first mark a1 on the stump (in other words, the proximal end) of the image of the true lumen 103 in the first angiographic image V1 displayed on the canvas A2. In accordance with the guidance, as illustrated in Fig. 24A, the operator arranges the first mark a1 on the first angiographic image V1 on the canvas A2.

In step S7A, the true lumen information acquisition unit 14A guides the operator to arrange the second mark a2 at a freely-selected position in a range in which the image of the true lumen 103 can be regarded as extending linearly with reference to the first mark a1, in the first angiographic image V1 displayed on the canvas A2. In accordance with the guidance, as illustrated in Fig. 24A, the operator arranges the second mark a2 on the image of the true lumen 103 appearing in the first angiographic image V1 on the canvas A2.

In step S8A, the true lumen information acquisition unit 14A calculates the BNV with the use of the first mark, the second mark, and the first position. The details are the same as those in step S8 of Fig. 4, and the processing may be performed by replacing "the first shaft axial vector of the imaging sensor 300" in step S8 with "the center axial vector of the true lumen 103 in the first angiographic image V1".

In step S10, the true lumen information acquisition unit 14A moves the first FPD 21 to the BNV (second position) calculated in step S8A, and guides the operator to capture an X-ray image. In accordance with the guidance, the operator moves the first FPD 21 to the BNV (second position) and captures an X-ray image to acquire the second angiographic image V2. The angiographic image acquisition unit 12 acquires the captured second angiographic image V2 from the blood vessel imaging device 20. As illustrated in Fig. 24B, the second angiographic image V2 includes an image of the distal end portion 401 of the wire catheter 400, an image of the guide wire 500 inserted into the wire catheter 400, an image (not illustrated) of the target blood vessel 100, and an image of the true lumen 103, which are captured from a direction different from that of the first angiographic image V1.

In step S11A, the true lumen information acquisition unit 14A displays the second angiographic image V2 on the canvas A2, and adjusts the position of the second angiographic image V2 in such a manner that the image of the distal end portion 401 of the wire catheter 400 is positioned at the center of the canvas A2.

In step S12A, the true lumen information acquisition unit 14A guides the operator to arrange the first mark b1 on the stump (in other words, the proximal end) of the image of the true lumen 103 in the second angiographic image V2 displayed on the canvas A2. In accordance with the guidance, as illustrated in Fig. 24B, the operator arranges the first mark b1 on the image of the true lumen 103 appearing in the second angiographic image V2 on the canvas A2.

In step S13, the true lumen information acquisition unit 14A substitutes 2 into the variable n used in the composite image output processing. N is a natural number. In step S14A, the true lumen information acquisition unit 14A guides the operator to arrange the second mark b2 at a freely-selected position distant from the stump of the image of the true lumen 103 in the distal direction. In accordance with the guidance, the operator arranges the second mark b2 on the image of the true lumen 103 appearing in the second angiographic image V2 on the canvas A2. In step S16, the true lumen information acquisition unit 14A adds 1 to the variable n.

In step S17, the true lumen information acquisition unit 14A determines whether the arrangement of the marks on the second angiographic image V2 (step S14A) has been completed for the target number of marks. When the variable n reaches the target number of marks (step S17: YES), the true lumen information acquisition unit 14A shifts the processing to step S18A. When the variable n has not reached the target number of marks (step S17: NO), the true lumen information acquisition unit 14A shifts the processing to step S14A and repeats the above-described processing.

In step S18A, the true lumen information acquisition unit 14A calculates the following (E1) and (E2) with the use of each coordinate of the first mark a1 and the second mark a2 in the first angiographic image V1, and each coordinate of the first mark b1 to the n-th mark bn in the second angiographic image V2.

(E1) True lumen vectors S2 to Sn: The true lumen vectors S2 to Sn correspond to the true lumen vectors S1 to Sn obtained in Figs. 4 and 5.

(E2) Vector P from the reference point (the distal end portion 401 of the wire catheter 400) of the reference device to the stump of the true lumen 103.

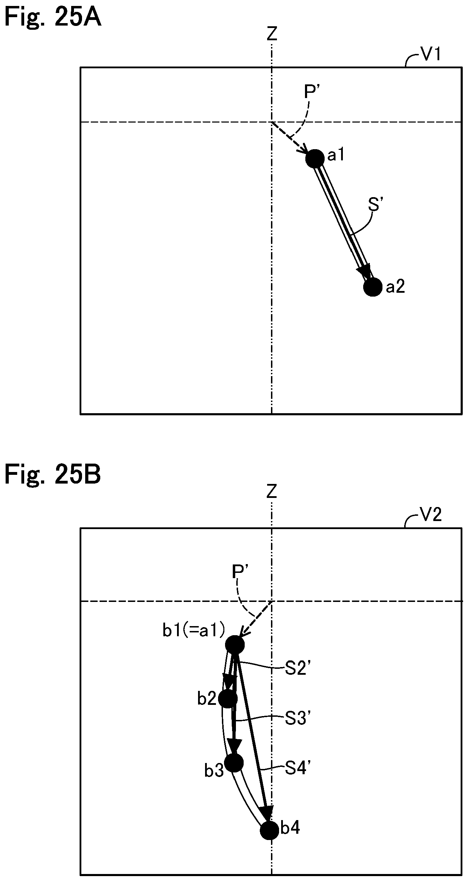

Figs. 25A and 25B are diagrams illustrating step S18A of the composite image output processing of the second embodiment. Fig. 25A is a diagram illustrating points on the first angiographic image V1, and Fig. 25B is a diagram illustrating points on the second angiographic image V2. In Figs. 25A and 25B, the Z-axis (Fig. 1: the direction in which the head 92 of the human body 90 is present) is located on the upper side of the plane. The first angiographic image V1 and the second angiographic image V2 of the second embodiment are images obtained by capturing the true lumen 103 from different angles, it can be said that the first mark b1 to the n-th mark bn (n = 4 in the illustrated example) in the second angiographic image V2 illustrated Fig. 25B are on the vector S' of the first angiographic image V1 illustrated in Fig. 25A. Therefore, the direction of the "(E1) true lumen vectors S2 to Sn" can be calculated using the inclination of the vector S' and the inclination of the vectors S2' to Sn'. The vector S' is a vector extending from the first mark a1 to the second mark a2. Further, the vector S2' is a vector extending from the first mark b1 to the second mark b2, and the vector Sn' is a vector extending from the first mark b1 to the n-th mark bn. The details of the method for calculating the direction of the "(E1) true lumen vectors S2 to Sn" are the same as those in step S18 of Fig. 5, and the processing may be performed by replacing the "position vectors P2 to Pn of the transducer 301" in step S18 with the "true lumen vectors S2 to Sn". Further, the true lumen information acquisition unit 14A calculates the lengths of the "(E1) true lumen vectors S2 to Sn" using Formula (4) described in step S18 of Fig. 5.

The direction of the "(E2) vector P from the reference point of the reference device to the stump of the true lumen 103" can be calculated using the inclination of the vector P' in the first angiographic image V1 and the inclination of the vector P' in the second angiographic image V2. The vector P' in the first angiographic image V1 is a vector extending from the reference point (the distal end portion 401 of the wire catheter 400) of the reference device in the first angiographic image V1 to the first mark a1. Further, the vector P' in the second angiographic image V2 is a vector extending from the reference point of the reference device to the first mark b1 (= a1) in the second angiographic image V2. The direction of the "(E2) vector P from the reference point of the reference device to the stump of the true lumen 103" can be calculated by applying the method for calculating the direction of the "position vectors P2 to Pn of the transducer 301" in step S18 of Fig. 5. The length of the "(E2) vector P from the reference point of the reference device to the stump of the true lumen 103" can be calculated using Formula (4) described in step S18 of Fig. 5.

In step S29, the true lumen information acquisition unit 14A stores the three dimensional position information of the true lumen acquired in step S18A in the storage unit inside the surgery assistance device 10. That is, in the example of the present embodiment, the three dimensional position information of the true lumen includes the directions of the true lumen vectors S2 to Sn and the lengths of the true lumen vectors S2 to Sn. In step S30, the image composition unit 16 superimposes and displays the true lumen on a captured image (angiographic image) at a freely-selected FPD position. Details are the same as those in step S30 of Fig. 5.

As described above, when the first angiographic image V1 and the second angiographic image V2 include the image of the true lumen 103, the three dimensional position information of the true lumen can be acquired without using the imaging sensor 300, as described in the second embodiment. Even in the second embodiment, the same effects as those of the first embodiment described above can be exhibited. According to the surgery assistance device 10A of the second embodiment, the true lumen information acquisition unit 14A can acquire the three dimensional position information of the true lumen by using the images of the medical devices and the images of the true lumen included in the first angiographic image and the second angiographic image.

<Third Embodiment>

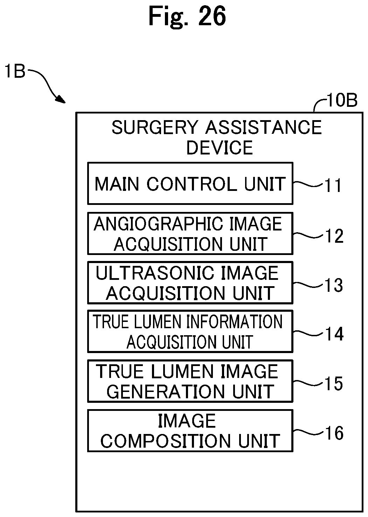

Fig. 26 is an explanatory diagram illustrating a configuration of a surgery assistance system 1B of a third embodiment. In the surgery assistance system 1B of the third embodiment, the surgery assistance device 10B is connected to a network, and the first angiographic image V1, the second angiographic image V2, the ultrasonic image IV1, and the ultrasonic image IVn are acquired from an external device via the network to acquire the three dimensional position information of the true lumen. Thereafter, the surgery assistance device 10B acquires the angiographic image VX arranged at a freely-selected imaging position, and generates the true lumen image VY including the image of the true lumen 103 at a position and posture corresponding to the angiographic image VX. The surgery assistance device 10B generates the composite image V by compositing the angiographic image VX and the true lumen image VY, and outputs the generated composite image V to the external device via the network. The surgery assistance device 10B may acquire the images V1, V2, IV1, and IVn from a storage medium such as a universal serial bus (USB) memory instead of the external device connected via the network, and output the composite image V to the storage medium.

As described above, the configuration of the surgery assistance system 1B can be variously changed, and the blood vessel imaging device 20, the display apparatus 30, the table 40, and the operation unit 50 may not be provided. In other words, the surgery assistance device 10B may be configured as an information processor or a server. Even in the third embodiment, the same effects as those of the first embodiment described above can be exhibited. Further, according to the surgery assistance system 1B of the third embodiment, it is possible to carry out a service of providing a composite image to an external device connected via a network.

<Modification of Embodiment>

The present invention is not limited to the above-described embodiments, and can be implemented in various modes without departing from the scope of the invention. For example, a part of the configuration implemented by hardware may be replaced by software, and conversely, a part of the configuration implemented by software may be replaced by hardware. In addition, for example, the following modifications are also possible.

[Modification 1]

In the first to third embodiments, the configurations of the surgery assistance systems 1, 1A, and 1B have been exemplified. However, the configuration of the surgery assistance system 1 can be variously changed. For example, the display apparatus 30 may be a monitor or a touch panel incorporated in the surgery assistance devices 10, 10A, and 10B. For example, the blood vessel imaging device 20 may have a configuration including a single FPD (in other words, a configuration not including the second FPD 25). For example, the surgery assistance system 1 may have another medical apparatus (e.g., a computerized tomography (CT) apparatus or a magnetic resonance imaging (MRI) apparatus) or the like not illustrated. At this time, the operation screen OS described with reference to Fig. 6A may include an image acquired by another medical apparatus.

[Modification 2]

The configurations of the surgery assistance devices 10, 10A, and 10B have been described in the first to third embodiments. However, the configuration of the surgery assistance device 10 can be variously changed. For example, the functions of the functional units included in the surgery assistance device 10 may be implemented by cooperation of a plurality of apparatuses connected via a network.

[Modification 3]

In the first to third embodiments, an example of the procedure of the composite image output processing has been described. However, the procedure of the composite image output processing described with reference to Figs. 4, 5, and 23 is merely an example, and can be variously changed. For example, the order of execution of the steps may be changed, at least some of the steps may be omitted, and other steps not described may be executed.

For example, the three dimensional position information of the true lumen may not be defined by the directions and the lengths of the true lumen vectors S1 to Sn. In this case, for example, the three dimensional position information of the true lumen can be defined by any information such as a set of point sequence coordinates forming the outer edge of the true lumen or a set of coordinates of feature points on the outer edge of the true lumen.

For example, the regeneration of the true lumen image VY and the re-output of the composite image Vb when the first FPD 21 is moved to the imaging position B (second imaging position) different from the imaging position A (first imaging position) may be omitted. Further, for example, when the first FPD 21 is moved to the imaging position B (second imaging position) different from the imaging position A (first imaging position), the image composition unit 16 may output both the composite image V at the first imaging position and the composite image Vb at the second imaging position to the canvas A2 in a visible manner. The mode in which both of them can be visually recognized can be appropriately determined, for example, by displaying them side by side or making it possible to refer to the history.

For example, the true lumen image generation unit 15 may make the image of the true lumen 103 included in the true lumen image VY translucent, or may make at least one of the hue/saturation/lightness of the image of the true lumen 103 a hue/saturation/lightness that is easy to distinguish from the imaging sensor 300 and the guide wire 500. In this way, the image of the true lumen 103 can be displayed without impairing the visibility of the medical device (the imaging sensor 300 and the guide wire 500). This adjustment may be automatically performed by image analysis of the angiographic image VX by the true lumen image generation unit 15, or may be changed in accordance with designation contents acquired from the operator.

For example, the image composition unit 16 may be capable of switching between display/non-display of the angiographic image XV and display/non-display of the true lumen image VY in the composite image V. In this way, usability of the surgery assistance devices 10, 10A, and 10B can be further improved.

For example, in the procedure of the composite image output processing of Figs. 4 and 5, the processing of step S19 may be included in step S3. To be more specific, "the true lumen information acquisition unit 14 guides the operator to move the first FPD 21 to the first position and capture the X-ray image" in step S3 may be changed to "the true lumen information acquisition unit 14 guides the operator to move the first FPD 21 to a first position (corresponding to the position $\alpha$ in step S19) at which the transducer 301 of the imaging sensor 300 and the guide wire 500 overlap (in other words, the transducer 301 of the imaging sensor 300 and the guide wire 500 intersect) to obtain the first angiographic image V1 (corresponding to the angiographic image V$\alpha$ in step S19) and to capture the X-ray image". In accordance with the guidance, the operator moves the first FPD 21 to the first position at which the first angiographic image V1 in which the transducer 301 and the guide wire 500 overlap is obtained, and acquires the first angiographic image V1 in which the transducer 301 and the guide wire 500 overlap. In this case, in step S21, the true lumen information acquisition unit 14 performs the directional calibration processing with the use of the first position of the first FPD 21. By changing the procedure of the composite image output processing as described above, step S19 can be omitted, and the efficiency of the composite image output processing can be improved.

[Modification 4]

The configurations of the surgery assistance devices 10, 10A, and 10B of the first to third embodiments and the configurations of the first to third modifications may be combined as appropriate. For example, the main control unit 11 may be configured to switch and execute the composite image output processing (Figs. 4 and 5) described in the first embodiment and the composite image output processing (Fig. 23) described in the second embodiment. The main control unit 11 may perform the switching in response to an instruction from the operator, or may automatically perform the switching by analyzing the first angiographic image V1 and the second angiographic image V2. For example, in the case of automatic switching, the main control unit 11 can execute the composite image output processing (Fig. 23) described in the second embodiment when the image of the true lumen 103 is included in the first angiographic image V1 and the second angiographic image V2, and can execute the composite image output processing (Figs. 4 and 5) described in the first embodiment when the image of the true lumen 103 is not included.

The present mode has been described above on the basis of the embodiments and the modifications; however, the embodiments according to the above modes are provided to facilitate understanding of the present mode and not to limit the mode. The present mode can be modified and improved without departing from the gist and the scope of the claims, and the present mode includes equivalents thereof. In addition, if the technical features are not described as essential in the present specification, the technical features may be appropriately deleted.

DESCRIPTION OF REFERENCE NUMERALS

1, 1A, 1B SURGERY ASSISTANCE SYSTEM
10, 10A, 10B SURGERY ASSISTANCE DEVICE
11 MAIN CONTROL UNIT
12 ANGIOGRAPHIC IMAGE ACQUISITION UNIT
13 ULTRASONIC IMAGE ACQUISITION UNIT
14, 14A TRUE LUMEN INFORMATION ACQUISITION UNIT
15 TRUE LUMEN IMAGE GENERATION UNIT
16 IMAGE COMPOSITION UNIT

20 BLOOD VESSEL IMAGING DEVICE
22 FIRST X-RAY TUBE DEVICE
23 FIRST C ARM
24 FIRST SUPPORT PORTION
26 SECOND X-RAY TUBE DEVICE
27 SECOND C ARM
28 SECOND SUPPORT PORTION
29 CONTROL UNIT
30 DISPLAY APPARATUS
31 MONITOR
32 ARM
40 TABLE
41 BED
42 EXPANSION/CONTRACTION PORTION
43 LEG PORTION
50 OPERATION UNIT
90 HUMAN BODY
91 HEART
92 HEAD
93 FEET
100 TARGET BLOOD VESSEL
102 FALSE LUMEN
103 TRUE LUMEN
200 SENSOR CATHETER
300 IMAGING SENSOR
301 TRANSDUCER
400 WIRE CATHETER
401 DISTAL END PORTION
500 GUIDE WIRE

**Claims**

1. A surgery assistance device comprising:

   a true lumen information acquisition unit that acquires three dimensional position information of a true lumen existing in a target blood vessel;
   a true lumen image generation unit that acquires an angiographic image of the target blood vessel from a flat panel detector (FPD) arranged at a first imaging position and generates a true lumen image representing the true lumen at a position and posture corresponding to the angiographic image by using position information of the first imaging position and three dimensional position information of the true lumen; and
   an image composition unit that generates a composite image by compositing the angiographic image and the true lumen image and outputs the composite image.

2. The surgery assistance device according to claim 1,

   wherein the three dimensional position information of the true lumen includes information on a width of the true lumen, and
   wherein the true lumen image generation unit generates a true lumen image representing the true lumen having a width corresponding to the three dimensional position information of the true lumen.

3. The surgery assistance device according to claim 1 or 2,

   wherein when the FPD is moved to a second imaging position different from the first imaging position and the target blood vessel is captured by the FPD, the true lumen image generation unit reacquires an angiographic image captured at the second imaging position, and regenerates a true lumen image representing the true lumen at a position and posture corresponding to the reacquired angiographic image by using position information of the second imaging position and three dimensional position information of the true lumen, and

wherein the image composition unit regenerates a composite image by compositing the reacquired angiographic image and the regenerated true lumen image, and outputs the composite image.

4. The surgery assistance device according to any one of claims 1 to 3, further comprising:

an angiographic image acquisition unit that acquires a first angiographic image captured by the FPD arranged at a first position and a second angiographic image captured by the FPD arranged at a second position different from the first position; and

an ultrasonic image acquisition unit that acquires an ultrasonic image of an inside of the target blood vessel captured by an ultrasonic sensor,

wherein the first angiographic image includes the ultrasonic sensor arranged at a first mark position within the target blood vessel, and a medical device different from the ultrasonic sensor arranged at a second mark position within the target blood vessel,

wherein the second angiographic image includes the ultrasonic sensor arranged at the first mark position within the target blood vessel,

wherein the ultrasonic image is an image captured in a state where the ultrasonic sensor is arranged at the first mark position,

wherein the ultrasonic image includes the target blood vessel and the medical device arranged at the second mark position within the target blood vessel, and

wherein the true lumen information acquisition unit acquires three dimensional position information of the true lumen by using position information of the first position, the first angiographic image, position information of the second position, the second angiographic image, and the ultrasonic image.

5. The surgery assistance device according to claim 4,

wherein the true lumen information acquisition unit acquires a position of the ultrasonic sensor by using images of the ultrasonic sensor included in the first angiographic image and the second angiographic image, associates a positional relation between the first angiographic image and the ultrasonic image by using images of the medical device included in the first angiographic image and the ultrasonic image,

the true lumen information acquisition unit acquires position information of the true lumen from the ultrasonic image, and

the true lumen information acquisition unit acquires three dimensional position information of the true lumen by using the acquired position of the ultrasonic sensor and position information of the true lumen in the ultrasonic image by the ultrasonic sensor.

6. The surgery assistance device according to any one of claims 1 to 3, further comprising an angiographic image acquisition unit that acquires a first angiographic image captured by the FPD arranged at a first position and a second angiographic image captured by the FPD arranged at a second position different from the first position,

wherein the first angiographic image includes the true lumen of the target blood vessel and a medical device arranged at a first mark position within the target blood vessel,

wherein the second angiographic image includes the true lumen of the target blood vessel and the medical device arranged at the first mark position within the target blood vessel, and

wherein the true lumen information acquisition unit acquires three dimensional position information of the true lumen by using an image of the medical device and an image of the true lumen, which are included in the first angiographic image and the second angiographic image.

7. A surgery assistance method comprising:

a true lumen information acquisition process that acquires three dimensional position information of a true lumen existing in the target blood vessel;

a true lumen image generation process that acquires an angiographic image of the target blood vessel from a flat panel detector (FPD) disposed at a first imaging position and generates a true lumen image representing the true lumen at a position and posture corresponding to the angiographic image by using position information of the first imaging position and three dimensional position information of the true lumen; and

an image composition process that generates a composite image by compositing the angiographic image and the true lumen image and outputs the composite image.

8. The surgery assistance method according to claim 7,

wherein the three dimensional position information of the true lumen includes information on a width of the true lumen, and

wherein the true lumen image generation process generates a true lumen image representing the true lumen having a width corresponding to the three dimensional position information of the true lumen.

9. The surgery assistance method according to claim 7 or 8,

wherein when the FPD is moved to a second imaging position different from the first imaging position and the target blood vessel is captured by the FPD, the true lumen image generation process reacquires an angiographic image captured at the second imaging position, and regenerates a true lumen image representing the true lumen at a position and posture corresponding to the reacquired angiographic image by using position information of the second imaging position and three dimensional position information of the true lumen, and

wherein the image composition process regenerates a composite image by compositing the reacquired angiographic image and the regenerated true lumen image, and outputs the composite image.

10. The surgery assistance method according to any one of claims 7 to 9, further comprising:

an angiographic image acquisition process that acquires a first angiographic image captured by the FPD arranged at a first position and a second angiographic image captured by the FPD arranged at a second position different from the first position; and

an ultrasonic image acquisition process that acquires an ultrasonic image of an inside of the target blood vessel captured by an ultrasonic sensor,

wherein the first angiographic image includes the ultrasonic sensor arranged at a first mark position within the target blood vessel, and a medical device different from the ultrasonic sensor arranged at a second mark position within the target blood vessel,

wherein the second angiographic image includes the ultrasonic sensor arranged at the first mark position within the target blood vessel,

wherein the ultrasonic image is an image captured in a state where the ultrasonic sensor is arranged at the first mark position,

wherein the ultrasonic image includes the target blood vessel and the medical device arranged at the second mark position within the target blood vessel, and

wherein the true lumen information acquisition process acquires three dimensional position information of the true lumen by using position information of the first position, the first angiographic image, position information of the second position, the second angiographic image, and the ultrasonic image.

11. The surgery assistance method according to claim 10, wherein the true lumen information acquisition process acquires a position of the ultrasonic sensor by using images of the ultrasonic sensor included in the first angiographic image and the second angiographic image, associates a positional relation between the first angiographic image and the ultrasonic image by using images of the medical device included in the first angiographic image and the ultrasonic image, acquires position information of the true lumen from the ultrasonic image, and acquires three dimensional position information of the true lumen by using the acquired position of the ultrasonic sensor and position information of the true lumen in the ultrasonic image by the ultrasonic sensor.

12. The surgery assistance method according to any one of claims 7 to 9, further comprising an angiographic image acquisition process that acquires a first angiographic image captured by the FPD arranged at a first position and a second angiographic image captured by the FPD arranged at a second position different from the first position,

wherein the first angiographic image includes the true lumen of the target blood vessel and a medical device arranged at a first mark position within the target blood vessel,

wherein the second angiographic image includes the true lumen of the target blood vessel and the medical device arranged at the first mark position within the target blood vessel, and

wherein the true lumen information acquisition process acquires three dimensional position information of the true lumen by using an image of the medical device and an image of the true lumen, which are included in the first angiographic image and the second angiographic image.

13. A computer program comprising:

a true lumen information acquisition step that acquires three dimensional position information of a true lumen existing in the target blood vessel;

a true lumen image generation step that acquires an angiographic image of the target blood vessel from a flat panel detector (FPD) disposed at a first imaging position and generates a true lumen image representing the true lumen at a position and posture corresponding to the angiographic image by using position information of the first imaging position and three dimensional position information of the true lumen; and

an image composition step that generates a composite image by compositing the angiographic image and the true lumen image and outputs the composite image.

14. The computer program according to claim 13,

wherein the three dimensional position information of the true lumen includes information on a width of the true lumen, and

wherein the true lumen image generation step generates a true lumen image representing the true lumen having a width corresponding to the three dimensional position information of the true lumen.

15. The computer program according to claim 13 or 14,

wherein when the FPD is moved to a second imaging position different from the first imaging position and the target blood vessel is captured by the FPD, the true lumen image generation step reacquires an angiographic image captured at the second imaging position, and regenerates a true lumen image representing the true lumen at a position and posture corresponding to the reacquired angiographic image by using position information of the second imaging position and three dimensional position information of the true lumen, and

wherein the image composition step regenerates a composite image by compositing the reacquired angiographic image and the regenerated true lumen image, and outputs the composite image.

16. The computer program according to any one of claims 13 to 15, further comprising:

an angiographic image acquisition step that acquires a first angiographic image captured by the FPD arranged at a first position and a second angiographic image captured by the FPD arranged at a second position different from the first position; and

an ultrasonic image acquisition step that acquires an ultrasonic image of an inside of the target blood vessel captured by an ultrasonic sensor,

wherein the first angiographic image includes the ultrasonic sensor arranged at a first mark position within the target blood vessel, and a medical device different from the ultrasonic sensor arranged at a second mark position within the target blood vessel,

wherein the second angiographic image includes the ultrasonic sensor arranged at the first mark position within the target blood vessel,

wherein the ultrasonic image is an image captured in a state where the ultrasonic sensor is arranged at the first mark position,

wherein the ultrasonic image includes the target blood vessel and the medical device arranged at the second mark position within the target blood vessel, and

wherein the true lumen information acquisition step acquires three dimensional position information of the true lumen by using position information of the first position, the first angiographic image, position information of the second position, the second angiographic image, and the ultrasonic image.

17. The computer program according to claim 16, wherein the true lumen information acquisition step acquires a position of the ultrasonic sensor by using images of the ultrasonic sensor included in the first angiographic image and the second angiographic image, associates a positional relation between the first angiographic image and the ultrasonic image by using images of the medical device included in the first angiographic image and the ultrasonic image, acquires position information of the true lumen from the ultrasonic image, and acquires three dimensional position information of the true lumen by using the acquired position of the ultrasonic sensor and position information of the true lumen in the ultrasonic image by the ultrasonic sensor.

18. The computer program according to any one of claims 13 to 15, further comprising an angiographic image acquisition step that acquires a first angiographic image captured by the FPD arranged at a first position and a second angiographic image captured by the FPD arranged at a second position different from the first position,

wherein the first angiographic image includes the true lumen of the target blood vessel and a medical device arranged at a first mark position within the target blood vessel,

wherein the second angiographic image includes the true lumen of the target blood vessel and the medical device arranged at the first mark position within the target blood vessel, and

wherein the true lumen information acquisition step acquires three dimensional position information of the true lumen by using an image of the medical device and an image of the true lumen, which are included in the first angiographic image and the second angiographic image.

# Fig. 1

EP 4 534 020 A1

# Fig. 2A

LAO

# Fig. 2B

RAO

# Fig. 2C

CRA

# Fig. 2D

CAU

Fig. 3A

Fig. 3B

EP 4 534 020 A1

# Fig. 4

START

PREPARE FOR IMAGING BY FPD — S1

PREPARE FOR IMAGING BY SENSOR — S2

MOVE FPD TO FIRST POSITION, ACQUIRE FIRST IMAGE — S3

POSITION TRANSDUCER AT CENTER OF SCREEN — S4

ARRANGE FIRST MARK ON TRANSDUCER — S5

ACQUIRE ULTRASONIC IMAGE IV1 — S6

ADVANCE SENSOR IN RANGE THAT CAN BE REGARDED AS STRAIGHT LINE, AND ARRANGE SECOND MARK ON FREELY-SELECTED TRANSDUCER — S7

CALCULATE BNV FROM FIRST AND SECOND MARKS AND FIRST POSITION — S8

PULL BACK TRANSDUCER TO POSITION OF FIRST MARK — S9

MOVE FPD TO BNV (SECOND POSITION), ACQUIRE SECOND IMAGE — S10

POSITION TRANSDUCER AT CENTER OF SCREEN — S11

ARRANGE FIRST MARK ON TRANSDUCER — S12

$n = 2$ — S13

ADVANCE SENSOR AND ARRANGE n-TH MARK ON TRANSDUCER — S14

ACQUIRE ULTRASONIC IMAGE IVn — S15

$n = n + 1$ — S16

$n$ = TARGET NUMBER OF MARKS ? — S17

NO

YES

1

# Fig. 5

( 1 )

CALCULATE BY USING MARK COORDINATES OF FIRST
IMAGE AND SECOND IMAGE
- POSITION VECTORS OF TRANSDUCER (P2 - Pn)
- TRANSDUCER AXIAL VECTORS (T1 - Tn) — S18

DETERMINE FPD POSITION $\alpha$ AT WHICH TRANSDUCER AND GW OVERLAP — S19

DISPLAY ULTRASONIC IMAGE IV1 — S20

PERFORM DIRECTIONAL CALIBRATION PROCESSING
(ASSOCIATE FPD DIRECTION WITH SENSOR DIRECTION) — S21

PERFORM SIZE CALIBRATION PROCESSING
(ASSOCIATE NUMBER OF PIXELS OF ULTRASONIC IMAGE WITH
ACTUAL DIMENSIONS) — S22

CALCULATE TRUE LUMEN VECTOR S1 — S23

n=2 — S24

DISPLAY ULTRASONIC IMAGE IVn — S25

CALCULATE TRUE LUMEN VECTOR Sn — S26

n=n+1 — S27

NO    n = TARGET NUMBER OF MARKS ? — S28

YES

STORE THREE DIMENSIONAL POSITION INFORMATION OF TRUE LUMEN — S29

SUPERIMPOSE AND DISPLAY TRUE LUMEN ON CAPTURED
IMAGE AT FREELY-SELECTED FPD POSITION — S30

END

## Fig. 6A

## Fig. 6B

Fig. 7A

Fig. 7B

Fig. 8A

Fig. 8B

Fig. 8C

Fig. 8D

Fig. 8E

# Fig. 9

## Fig. 10A

## Fig. 10B

# Fig. 11

Fig. 12A

Fig. 12B

# Fig. 13

## Fig. 14

# Fig. 15

# Fig. 16

Fig. 17A

21(A)

VnA

Sn

Spn

VY(VY′)

Fig. 17B

Oc

VY

Xc

Zp

Spn(x,y)

θp

Spn′(x′,y′)

Yc

Fig. 17C

VY

21(A)

VnA

θ

Spn
(Spn$_{length}$)

Sn(Sn$_{length}$)

Fig. 18A

Fig. 18B

Fig. 19A

Fig. 19B

Fig. 20A

300

500

Vc(VX+VY)

103

Fig. 20B

300

500

Vd(VX+VY)

103

Fig. 21

Fig. 22

## Fig. 23

START

PREPARE FOR IMAGING BY FPD ~S1

MOVE FPD TO FIRST POSITION, ACQUIRE FIRST IMAGE ~S3A

POSITION REFERENCE DEVICE AT CENTER OF SCREEN ~S4A

ARRANGE FIRST MARK ON STUMP OF TRUE LUMEN ~S5A

ARRANGE SECOND MARK AT FREELY-SELECTED POSITION IN RANGE IN WHICH TRUE LUMEN CAN BE REGARDED AS STRAIGHT LINE ~S7A

CALCULATE BNV FROM FIRST AND SECOND MARKS AND FIRST POSITION ~S8A

MOVE FPD TO BNV (SECOND POSITION), ACQUIRE SECOND IMAGE ~S10

POSITION REFERENCE DEVICE AT CENTER OF SCREEN ~S11A

ARRANGE FIRST MARK ON STUMP OF TRUE LUMEN ~S12A

n=2 ~S13

ARRANGE n-TH MARK IN DIRECTION DISTANT FROM STUMP OF TRUE LUMEN ~S14A

n=n+1 ~S16

NO ← n = TARGET NUMBER OF MARKS ? ~S17

YES

CALCULATE BY USING MARK COORDINATES OF FIRST IMAGE AND SECOND IMAGE
- TRUE LUMEN VECTORS (S2 - Sn)
- VECTOR (P) FROM REFERENCE POINT OF REFERENCE DEVICE TO STUMP OF TRUE LUMEN ~S18A

STORE THREE DIMENSIONAL POSITION INFORMATION OF TRUE LUMEN ~S29

SUPERIMPOSE AND DISPLAY TRUE LUMEN ON CAPTURED IMAGE AT FREELY-SELECTED FPD POSITION ~S30

END

Fig. 24A

Fig. 24B

Fig. 25A

Fig. 25B

# Fig. 26

1B

10B

SURGERY ASSISTANCE
DEVICE

| MAIN CONTROL UNIT | 11 |

| ANGIOGRAPHIC IMAGE
ACQUISITION UNIT | 12 |

| ULTRASONIC IMAGE
ACQUISITION UNIT | 13 |

| TRUE LUMEN INFORMATION
ACQUISITION UNIT | 14 |

| TRUE LUMEN IMAGE
GENERATION UNIT | 15 |

| IMAGE
COMPOSITION UNIT | 16 |

## INTERNATIONAL SEARCH REPORT

| International application No. |
| --- |
| **PCT/JP2022/028524** |

### A. CLASSIFICATION OF SUBJECT MATTER

*A61B 8/12*(2006.01)i; *A61B 34/20*(2016.01)i; *A61B 6/00*(2006.01)i; *A61B 6/03*(2006.01)i; *A61B 6/12*(2006.01)i
FI:　A61B6/03 360Q; A61B6/00 331A; A61B6/00 350P; A61B6/12; A61B8/12; A61B34/20

According to International Patent Classification (IPC) or to both national classification and IPC

### B. FIELDS SEARCHED

Minimum documentation searched (classification system followed by classification symbols)

A61B8/12; A61B34/20; A61B6/00; A61B6/03; A61B6/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2022-55170 A (TERUMO CORP.) 07 April 2022 (2022-04-07) paragraphs [0013]-[0073], etc. in particular, paragraphs [0014], [0015], [0020], [0022], [0031], [0032], [0037], [0041], [0042], etc. | 1-3, 6-9, 12-15, 18 |
| Y | paragraphs [0013]-[0073], etc. in particular, paragraphs [0014], [0015], [0020], [0022], [0031], [0032], [0037], [0041], [0042], etc. | 4-5, 10-11, 16-17 |
| Y | CN 108013934 A (SHANGHAI UNITED IMAGING HEALTHCARE CO., LTD.) 11 May 2018 (2018-05-11) paragraphs [0049], [0050] | 4-5, 10-11, 16-17 |
| A | JP 2007-136164 A (TOSHIBA CORP.) 07 June 2007 (2007-06-07) entire text, all drawings | 1-18 |
| A | US 5771895 A (SLAGER, Cornelis J.) 30 June 1998 (1998-06-30) entire text, all drawings | 1-18 |
| A | JP 2013-85652 A (TOSHIBA CORP.) 13 May 2013 (2013-05-13) entire text, all drawings | 1-18 |

☑ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **07 September 2022** | **20 September 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2022/028524**

## C.    DOCUMENTS CONSIDERED TO BE RELEVANT

| Category* | Citation of document, with indication, where appropriate, of the relevant passages | Relevant to claim No. |
|---|---|---|
| A | JP 2020-520759 A (KONINKLIJKE PHILIPS N.V.) 16 July 2020 (2020-07-16)<br>entire text, all drawings | 1-18 |
| A | JP 2013-233413 A (TOSHIBA CORP.) 21 November 2013 (2013-11-21)<br>entire text, all drawings | 1-18 |

Form PCT/ISA/210 (second sheet) (January 2015)

**INTERNATIONAL SEARCH REPORT**
**Information on patent family members**

International application No.

**PCT/JP2022/028524**

| Patent document cited in search report | | | Publication date (day/month/year) | Patent family member(s) | | | Publication date (day/month/year) |
|---|---|---|---|---|---|---|---|
| JP | 2022-55170 | A | 07 April 2022 | (Family: none) | | | |
| CN | 108013934 | A | 11 May 2018 | (Family: none) | | | |
| JP | 2007-136164 | A | 07 June 2007 | US | 2007/0092067 | A1 | |
| | | | | entire text, all drawings | | | |
| | | | | CN | 1973772 | A | |
| US | 5771895 | A | 30 June 1998 | WO | 1997/028743 | A1 | |
| JP | 2013-85652 | A | 13 May 2013 | US | 2013/0259336 | A1 | |
| | | | | entire text, all drawings | | | |
| | | | | WO | 2013/058114 | A1 | |
| | | | | CN | 103327899 | A | |
| JP | 2020-520759 | A | 16 July 2020 | US | 2020/0085398 | A1 | |
| | | | | entire text, all drawings | | | |
| | | | | WO | 2018/215499 | A1 | |
| | | | | EP | 3406195 | A1 | |
| | | | | CN | 110650686 | A | |
| JP | 2013-233413 | A | 21 November 2013 | US | 2013/0266123 | A1 | |
| | | | | entire text, all drawings | | | |
| | | | | CN | 103356212 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6770655 B
- JP 2013233413 A

- JP 2021034980 W